# EUROPEAN PATENT SPECIFICATION

(11) **EP 0 358 536 B1**
(45) Date of publication and mention of the grant of the patent: **08.05.1996**
(21) Application number: 89401845.6
(22) Date of filing: 28.06.1989
(51) Int. Cl.: C07H 19/167, A61K 31/70

(54) **Novel S-adenosyl methionine decarboxylase inhibitors**
S'-adenosyl-methionin-decarboxylase-Inhibitoren
Inhibiteurs de la S'-adénosylméthionedécarboxylase

(30) Priority: 21.07.1988 EP 88401896
(43) Date of publication of application: 14.03.1990
(73) Proprietor: MERRELL PHARMACEUTICALS INC., Cincinnati, Ohio 45215-6300 (US)
(72) Inventor: Casara, Patrick, F-67117 Ittenheim (FR); Danzin, Charles, F-67000 Strasbourg (FR)
(74) Representative: Gillard, Marie-Louise

(56) References cited:
- JOURNAL OF MEDICINAL CHEMISTRY, vol. 19, no. 5, May 1976, pages 684-691; C.-D. CHANG et al.: "Analogues of S-adenosylhomocysteine as potential inhibitors of biological transmethylation. Synthesis of analogues with modifications at the 5'-thioether linkage"
- JOURNAL OF MEDICINAL CHEMISTRY, vol. 23, no. 2, February 1980, pages 121-127, American Chemical Society; M. PANKASKIE et al.: "Inhibitors of polyamine biosynthesis. 8. Irreversible inhibition of mammalian S-adenosyl-L-methionine decarboxylase by substrate analogues"
- JOURNAL OF MEDICINAL CHEMISTRY, vol. 25, no. 5, May 1982, pages 550-556, American Chemical Society; M. KOLB et al.: "Synthesis and biochemical properties of chemically stable product analogues of the reaction catalyzed by S-adenosyl-L-methionine decarboxylase"
- CHEMICAL ABSTRACTS, vol. 100, no. 13, 26th March 1984, pages 698-699, abstract no. 103834n, Columbus, Ohio, US; M. DAVIS et al.: "The synthesis of the N-methyl analog of S-adenosylmethionine: NMR observation of diastereomers", & AUST. J. CHEM. 1983, 36(8), 1623-7
- BIOCHEMISTRY, vol. 21, no. 7, 30th March 1982, pages 1535-1541, American Chemical Society; C.S.G. PUGH et al.: "Effects of S-adenosylhomocysteine analogues on vaccinia viral messenger ribonucleic acid synthesis and methylation"
- JOURNAL OF ORGANIC CHEMISTRY, vol. 53, no. 21, 14th October 1988, pages 4952-4961, American Chemical Society; A.A. MINNICK et al.: "General synthetic approach to stable nitrogen analogues of S-adenosylmethionine"

## Description

This invention relates to novel chemical compounds useful as S-adenosylmethionine decarboxylase inhibitors, to the processes useful for their preparation and to their use in the treatment of a variety of condition and disease states associated with a rapid proliferation of cell growth.

Some S-adenosylmethionine decarboxylase inhibitors have already been described in the following documents :
- J. Med. Chem., Vol. 23, N° 2, 1980, pages 121-127
- J. Med. Chem., Vol. 25, N° 5, 1982, pages 550-556
- Chem. Abs. 100, N° 13, 1984, 103834n
- J. Org. Chem., Vol. 53, N° 21, 1988, pages 4952-5961.

On the other hand, the synthesis of S-adenosylhomocysteine decarboxylase inhibitors have been disclosed in Biochemistry, Vol. 21, N°7, 1982, pages 1535-1541, and also their activity as inhibitors of biological transmethylation in J. Med. Chem., Vol. 19, N° 5, 1976, pages 684-691.

More specifically, this invention relates to compounds of the formula 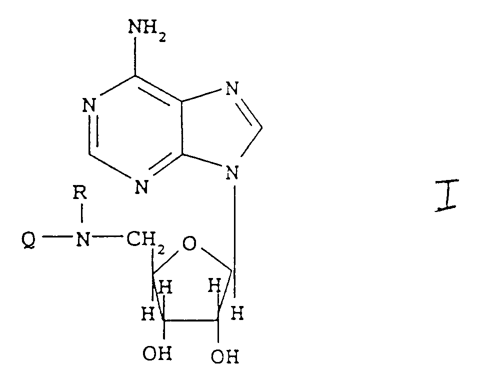
and the pharmaceutically acceptable salts thereof, wherein
R is hydrogen or a C₁₋₇ alkyl,
Q represents a moiety of one of the formulae Ia or Ie : 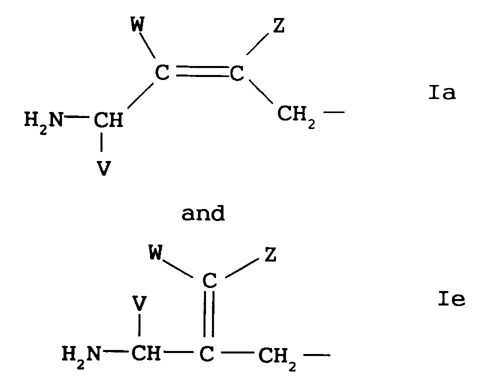
wherein
V is H or -COOH,
W is H, F, Cl or Br,
Z is H, F, Cl or Br.

In those instances wherein R is other than H, the C₁₋₇ alkyl moiety is preferably methyl and ethyl but all the straight, branched-chain and cyclized manifestations are included with methyl being preferred and ethyl, propyl, isopropyl, butyl, t-butyl, pentyl, hexyl, cyclohexylmethyl and the like being especially included. In those instances wherein Q is representative of formula Ie, it is preferred that said compounds be in their cis-configuration rather than their trans-configuration. Preferred compounds are those of formula I wherein Q is Ie, are those wherein W and Z are H and V is H or COOH, and R is methyl or ethyl.

Illustrative examples of pharmaceutically acceptable salts of the compounds of this invention are those formed with inorganic acids preferably with hydrochloric, hydrobromic, sulfuric or phosphoric acids and with organic acids such as methane sulfonate, salicylic, maleic, malonic, tartaric, citric and ascorbic acids. These salts may be prepared by standard techniques and procedures well known in the art.

In essence, the preparation of the compounds of formula I may be effected by techniques and chemical processes analogously known in the art; the choice of the specific route being dependent upon the usual factors in pharmaceutical research institutions such as availability and cost of starting materials, time and difficulties in separation and purification of intermediates and final compounds and such other factors well known and generally appreciated by those of ordinary skill in the art.

In general the preparation of the compounds of formula I may be depicted by the reaction sequence of reaction scheme A, as follows: 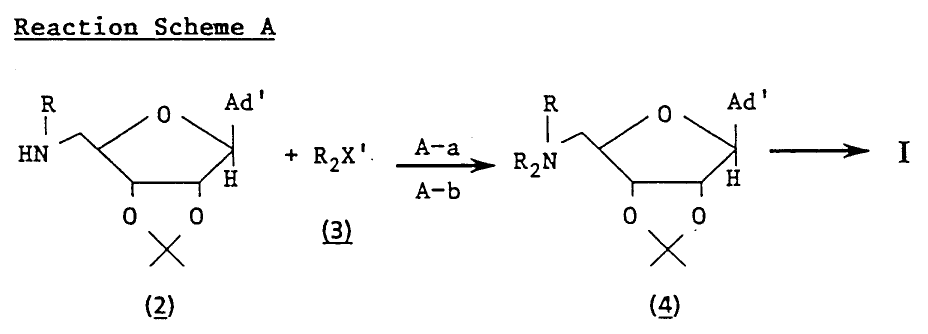
wherein Ad' is adeninyl of the formula 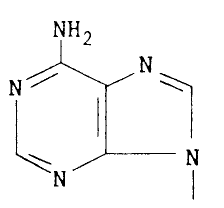
R₂X' is a reactant of the following formulae (3a or 3e). 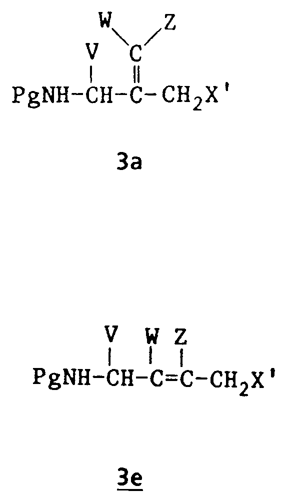
wherein Pg is an N-protecting group, preferably t-butoxycarbonyl (Boc) or phthalimido (Pht) (in which case, of course, the H of the PgNH moiety is not present) and R, V, W, and Z are as defined in Formula I, and X' is OTF (triflate) or chloro, bromo or iodo, (R₂, of course, being the moieties of 3a or 3e attached to the X' moiety), with the exception that in appropriate cases V may be a reaction-protection derivative of the COOH function, preferably a t-butoxy derivative, and Ac is an acyl moiety, preferably acetate, and R₃ is t-butyl.

In effecting the condensation of reactants 2 and 3 when X' represents a halide conditions A-a are utilized wherein equimolar quantities of the reactants are reacted together in the presence of a base, (preferably potassium carbonate), in a basic solvent, (preferably acetonitrile), at temperatures of about 30°C to 80°C. When conditions A-b are utilized, i.e., when X' is a triflate, the reactants are heated together at about 30°C to 80°C in the presence of a base, (preferably triethylamine), in a basic solvent, (preferably dimethylformamide). Removal of the N-protecting groups is readily effected by standard techniques, e.g., treatment with 1N sulfuric acid at room temperature for 24-48 hours followed by treatment with an alcohol (preferably ethanol) at about 0°C when the protecting group is t-butoxycarbonyl, and when the protecting group is phthalimido, removal is effected using an ethanolic solution of a hydrazine (using classical techniques) the latter being used when R₂ contains a fluoro atom. Removal of the isopropylidene protecting group of the ribofuranosyl moiety is easily effected by hydrolysis at room temperature, (preferably using 1N sulfuric acid), generally simultaneously with the N-protecting groups. Isolation and purification of the intermediate and final products of reaction scheme A is effected by standard techniques, e.g., recrystallization, HPLC, flash chromatography (on silica gel) and the like.

The preparation of the intermediates required for the condensation of reaction scheme A, i.e., those intermediates defined for R₂X', may be effected by the use of analogously known procedures such as those outlined in the below described generic processes which are illustrated in the below particularized examples.

In those instances wherein R₂X' represents subgeneric group 3e, the reaction proceeds under A-a conditions wherein X' is preferably chloro and the N-protecting group is Boc, the appropriate V, W, Z-substituted-N-protected-4-chloro-2-butene-1-amine may conveniently be prepared by the following reaction scheme. 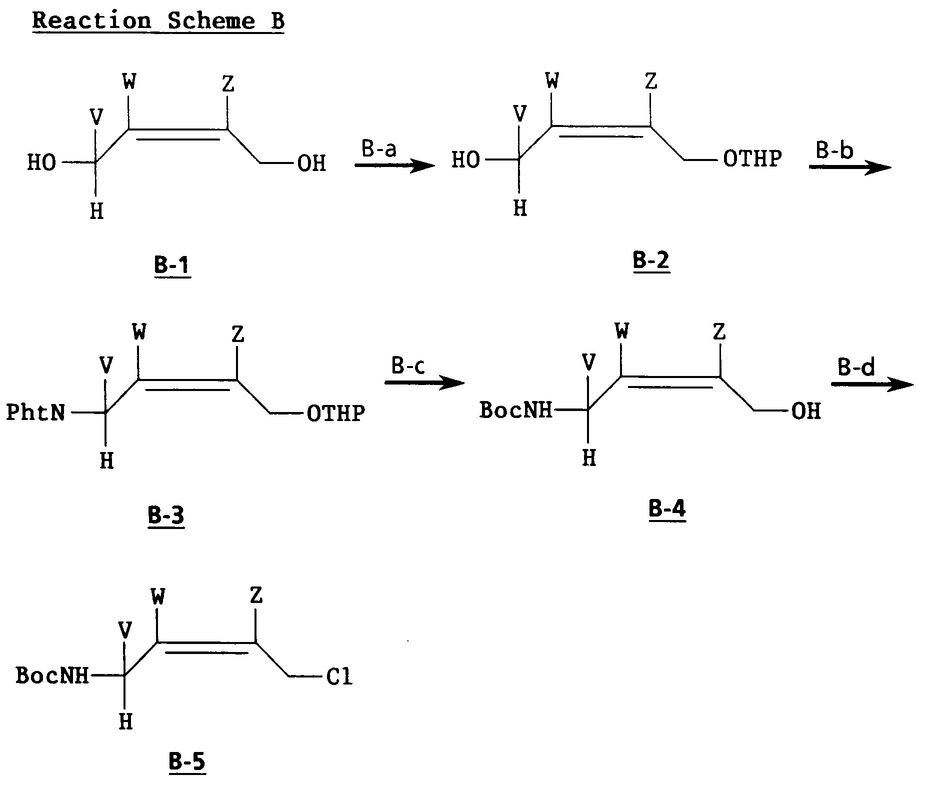
wherein the protecting groups (Pht and Boc) and V, W and Z are as previously defined and (THP) is tetrahydropyran.

In step B-a the diol is reacted with dihydropyran in the presence of catalytic quantities of pyridinium-p-toluene sulfonate at about 0°C in an anhydrous solvent (or mixture) (e.g., CH₂Cl₂:THF; 2:1) for about 24-48 hours. Conversion of B-2 to B-3 is initiated by a Mitsunobu-type intermolecular dehydration reaction on treatment with diethylazodicarboxylate (DEAD) and triphenylphosphine under mild neutral conditions under an inert atmosphere (nitrogen) at about 0°C in an anhydrous solvent (e.g., THF) in the presence of phthalimide; the reaction continuing at room temperature for about 12 hours.

The resulting B-3 products, upon treatment with hydrazine hydrate in ethanol at reflux for about 12 hours, to remove the phthalimido and THP protecting groups and the free amine is re-protected with di-t-butyldicarbonate by refluxing in dichloromethane. The alcohols (B-4) are converted to their chlorides by reaction with mesylchloride under basic conditions (TEA) in an anhydrous solvent, preferably dichloromethane. These cis products of Formula 3e, after purification, generally using flash chromatographic techniques on silica gel, are ready for condensation with the reactants of Formula 2, according to the techniques described for reaction scheme A.

In those instances wherein it is desired to prepare the trans-configuration of compounds of 3-e, it is preferred to utilize a W, Z-V-substituted N-protected trans-1-bromo-4-amino-2-butene, (i.e., 3-e), the reactants are readily prepared by reacting a V-W, Z-substituted trans-1-bromo-4-amino-2-butene with potassium phthalimide in anhydrous DMF at about 50°C for 24 hours according to standard procedures well known in the art. The necessary R₂X' reactants of the class 3-c are readily prepared from the appropriate W, Z, V₂-substituted α,α-dichloroxylene wherein the compound is subjected to a displacement reaction with potassium phthalimide to form an α-phthalimido-α'-chloroxylene by heating the reactants at about 50°C for about 24 hours in anhydrous DMF and the so-formed compound is purified by the usual techniques of flash chromatography from silica gel. Starting from the appropriately V, W, Z-substituted 3-chloro-2-chloromethyl-1-propene the desired R₂X' reactants of class 3-a may similarly be prepared by the foregoing described displacement reaction with potassium phthalimide by heating the reactants at about 50°C for about 24 hours in anhydrous dimethylfluoromethane followed by purification with the usual techniques, e.g., flash chromatography. In those instances wherein the particular V, W, Z-substituted reactant is not a known compound, such compounds may be prepared by techniques and procedures well understood and known in the art.

In addition to the specific examples described below, chemistry for the preparation of cis-5'-(4-amino-4-carboxy-2-butenyl)methyladenosine may be analogously derived from Tolman and Sedmera's article (Tetrahedron Letters, Vol. 29, No. 47, pp. 6183-6184, 1988) "Unsaturated Amino Acids: Synthesis of Trans-3,4-Didehydro Analogues of L-Ornithine and L-Arginine". The application of this chemistry is schematically represented by the following reaction scheme. 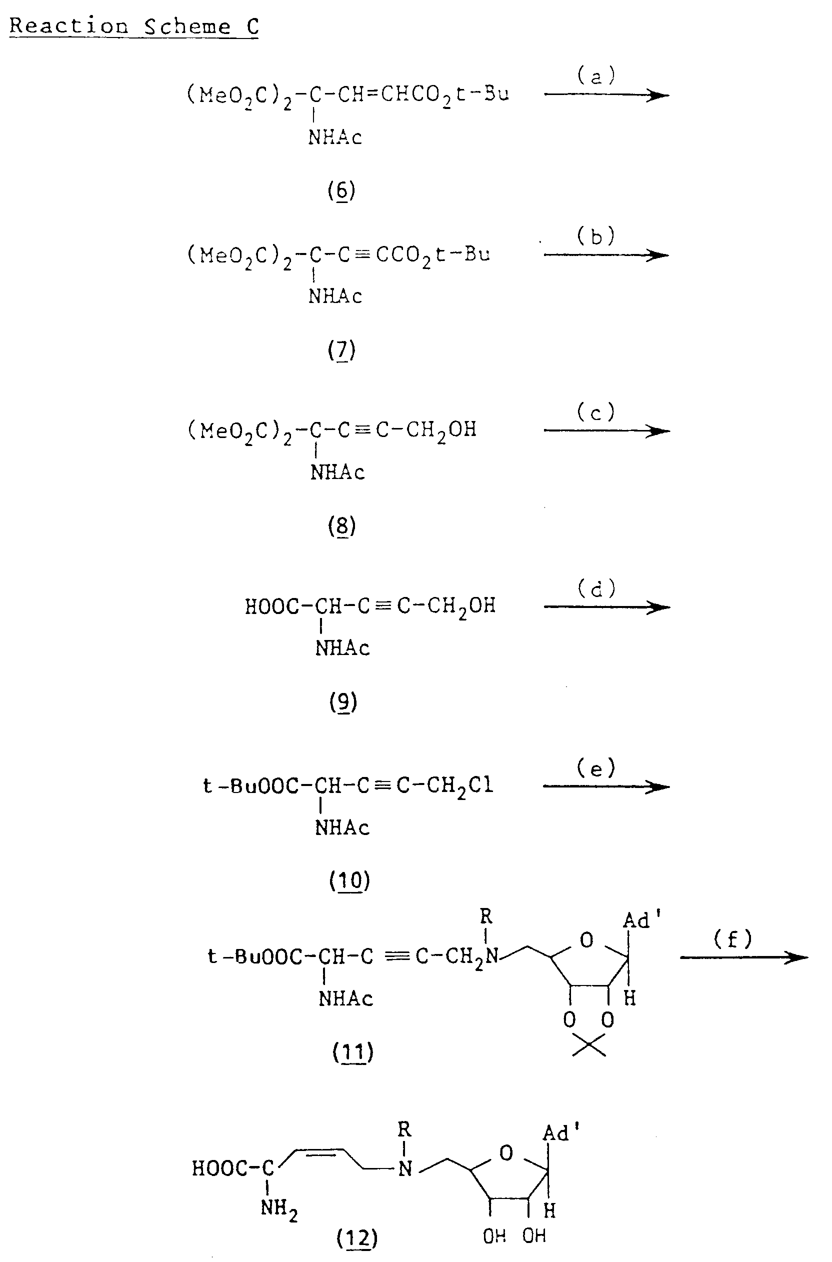
In effecting the foregoing reaction scheme step (a) involves the dibromination of (6) by reaction with bromine, said reaction being placed in a suitable solvent (e.g., CCl₄) at room temperature. The resulting dibromo analog is debrominated by reaction with potassium t-butoxide in tetrahydrofuran or with an amine such as DBU. The so-obtained compound (7) is sequentially treated with (₁) trifluoroacetic acid at 25°C for 20 minutes, (₂) treated with thionyl chloride at 25°C for 3 hours, and (₃) treated with DiBal in tetrahydrofuran at -30°C for 1 hour to produce compound (8). Step (c) involves the sequential treatment of (8) with a base (e.g., NaOH/H₂O in tetrahydrofuran for 20 minutes), followed by treatment with diluted HCl at 50°C to produce compound (9). This compound is treated with isobutylene, in the presence of catalytic amounts of sulfuric acid and the resulting alcohol is converted to its corresponding chloride by treatment with mesyl chloride to produce compound (10). This compound is then subjected to reaction with the adenosine derivatives of formula (2) according to the procedure of Reaction Scheme A (wherein compound (10) corresponds to R₂X' with X' being chloro) to produce a compound analogously corresponding to compounds 4 [i.e., compound (11)].

The resulting triple-bond-containing compound is partially reduced using hydrogenation in the presence of a Lindlar catalyst (H₂/PdSO₄) and the resulting butene is treated with sulfuric acid (to remove the t-butoxide and isopropylidene protecting groups). The final step is to subject the so-produced penultimate compound to acylase I (Merck) at a pH of 7.2 at 37°C to remove the N-protecting acyl moiety to produce a desired compound (12), e.g., cis-5'-deoxy-5'-(4-amino-4-carboxy-2-butene )methylaminoadenosine.

The present invention also relates to the intermediates as hereinabove defined.

The following examples illustrate the preparation of the necessary intermediates and final products of this invention.

### EXAMPLE 1

### Preparation of CIS-5'-DEOXY-5'-(4-AMINO-2-BUTENYL)METHYLAMINOADENOSINE

### Step A: CIS-4-TETRAHYDROPYRANYLOXY-2-BUTENE-1-OL

Dihydropyrane (9.1 ml, 100 mmol) was added dropwise to a cooled (0°C) solution of 2-butene-1,4-diol (8.8 g, 100 mmol) and pyridinium paratoluenesulfonate (25 g, 100 mmol) in anhydrous dichloromethane:tetrahydrofuran (2:1). The mixture was stirred two days at 0°C then concentrated *in vacuo*. The residue was purified by flash chromatography on silica gel (ethyl acetate:hexane 3:7) to give 8.3 g of the title compound (49%).

### Step B: CIS-1-PHTHALIMIDO-4-TETRAHYDROPYRANYLOXY-2-BUTENE

Under a nitrogen atmosphere diethylazodicarboxylate (1.6 ml, 10 mmol) was added to a cooled (0°C) solution of cis-4-tetrahydropyranyloxy-2-butene-1-ol (1.7 g, 10 mmol), triphenyl phosphine (2.2 g, 10 mmol) and phthalimide (1.47 g, 10 mmol) in anhydrous tetrahydrofuran (50 ml). When the addition was completed (5 min) the reaction mixture was allowed to warm at room temperature and was stirred 12 h. Then the mixture was concentrated *in vacuo*, diluted with ethyl acetate (200 ml) and washed with brine (150 ml). After usual work-up (the aqueous phase was extracted three times with 100 ml portions of ethyl acetate, the organic phase was dried over magnesium sulfate, filtered and concentrated *in vacuo*) the product was purified by flash chromatography on silica gel (ethyl acetate:hexane; 2:8) to give 1.9 g of the title compound (64%).

### Step C: CIS-TERTIOBUTOXYCARBONYL-4-HYDROXY-2-BUTENYL-1-AMINE

A solution of cis-1-phthalimido-4-tetrahydropyranyloxy-2-butene (1.9 g, 6.3 mmol) and hydrazine hydrate (0.35 ml, 6.9 mmol) in ethanol (20 ml) was heated under reflux 12 hours. Then the mixture was concentrated *in vacuo*, diluted with 1N hydrochloric acid (20 ml) and heated under reflux for two hours. Then the phthalylhydrazide was filtered off and the filtrate was concentrated *in vacuo*. The residue was taken in dichloromethane (100 ml) neutralized with triethylamine (pH 8.9) and a solution of ditertiobutyldicarbonate (1.65 g, 7.5 mmol) in dichloromethane (5 ml) was added. The mixture was heated under reflux overnight and, after usual work-up, the product was obtained by flash chromatography on silica gel (ethyl acetate: hexane; 25:75) (0.8 g, 74%).

### Step D: CIS-N-TERTIOBUTOXYCARBONYL-4-CHLORO-2-BUTENYL-1-AMINE

Mesyl chloride (0.6 ml, 7.6 mmol) was added to a cooled (0°C) solution of cis-tertiobutoxycarbonyl-4-hydroxy-2-butenyl-1-amine (1.3 g, 7 mmol) and triethylamine (1.1 ml, 7.6 mmol) in anhydrous dichloromethane (30 ml). The mixture was stirred overnight and, after usual work-up, the title product was purified by flash chromatography on silica gel (ethyl acetate: hexane; 2:8) (0.8 g, 57%).

### Step E: CIS-5'-DEOXY-5'(N-TERTIOBUTOXYCARBONYL-4-AMINO-2-BUTENYL)-METHYL-AMINO-2',3'-ISOPROPYLIDENEADENOSINE

A solution of cis-N-tertiobutoxycarbonyl-4-chloro-2-butenyl-1-amine (0.6 g, 3 mmol), 5'-deoxy-5'-methylamino-2',3'-isopropylideneadenosine (0.97 g, 3 mmol), potassium carbonate (0.42 g, 3 mmol) and sodium iodide (0.05 g, 0.3 mmol) in acetonitrile (20 ml) was heated under reflux overnight. Then the mixture was diluted with ethyl acetate, washed with brine and dried over magnesium sulphate. Then the product was purified by flash chromatography on silica gel (diethyl amine:chloroform; 2:98) (1.1 g, 55%).

### Step F: CIS-5'-DEOXY-5'(4-AMINO-2-BUTENYL)METHYLAMINOADENOSINE

A solution of cis-5'-deoxy-5'[(N-tertiobutoxycarbonyl-4-amino-2-butenyl)methyl-amino]-2',3'-isopropylideneadenosine (0.9 g, 1.8 mmol) in 1N sulphuric acid (5 ml) was left two days at room temperature. Then the mixture was diluted with ethanol (200 ml) and cooled (0°C) overnight. The precipitate was filtered off, dissolved in the minimum amount of water and then re-precipitated with ethanol (200 ml). This procedure was repeated twice to give the title compound (0.5 g), mp: 260°C decomposed.

### EXAMPLE II

### Preparation of TRANS-5'-DEOXY-5'-(4-AMINO-2-BUTENYL)METHYLAMINOADENOSINE

### Step A: TRANS-1-BROMO-4-PHTHALIMIDO-2-BUTENE

A mixture of trans-1,4-dibromo-2-butene (6.4 g, 30 mmol) and potassium phthalimide (5.6 g, 30 mmol) in anhydrous dimethyl formamide (200 ml) was heated at 50°C for 24 h. Then the reaction mixture was concentrated in vacuo, dissolved in ethyl acetate, washed with brine and the pure title product was obtained by flash chromatography on silica gel (ethyl acetate: hexane; 15:85) (3.2 g, 40%).

### Step B: TRANS-5'-DEOXY-5'-(4-PHTHALIMIDO-2-BUTENYL)METHYLAMINO-2',3'-ISOPROPYLIDENEADENOSINE

A mixture of trans-1-bromo-4-phthalimido-2-butene (2 g, 7.5 mmol), potassium carbonate (1.6 g, 11.5 mmol) and 5'-deoxy-5'-methylamino-2',3'-isopropylideneadenosine (2.4 g, 7.5 mmol) in anhydrous acetonitrile (100 ml) was heated under reflux overnight. Then the mixture was concentrated *in vacuo*, dissolved in dichloromethane, filtered and purified by flash chromatography on silica gel (chloroform: diethylamine; 98:2) to afford the title compound (1.25 g, 33%).

### Step C: TRANS-5'-DEOXY-5'-(4-TERTIOBUTOXYCARBONYLAMINO-3-BUTENYL)-METHYLAMINO-2',3'-ISOPROPYLIDENEADENOSINE

A mixture of trans-5'-deoxy-5'-(4-phthalimido-2-butenyl)-methylamino-2',3'-isopropylideneadenosine (1 g, 2 mmol) and hydrazine hydrate (0.1 ml, 2 mmol) in absolute ethanol was heated under reflux overnight. Then the mixture was concentrated *in vacuo*, dissolved in water (30 ml) and the pH was adjusted to 4 with glacial acetic acid and cooled to 0°C. Then the mixture was filtered off and the filtrate neutralized with triethylamine to pH 9 and concentrated *in vacuo*. Then the residue was dissolved in dichloromethane, and ditertiobutyldicarbonate (0.45 g, 2 mmol) was added. The mixture was heated under reflux overnight and, after usual work-up, the product was purified by flash chromatography on silica gel (diethylamine:dichloromethane; 2:98) to give the title compound (0.5 g, 51%).

### Step D: TRANS-5'-DEOXY-5'-(4-AMINO-2-BUTENYL)METHYLAMINOADENOSINE

A suspension of trans-5'-deoxy-5'-(4-tertiobutoxycarbonylamino-2-butenyl)methylamino-2',3'-isopropylideneadenosine (0.4 g, 0.96 mmol) in 1N sulphuric acid (3 ml) was stirred 2 days at room temperature. Then the mixture was diluted with absolute ethanol (100 ml) and cooled at 0°C overnight. The product was filtered off, dissolved in the minimum amount of water and precipitated with ethanol (100 ml). This procedure was repeated twice to afford the title compound (0.16 g). mp: 250-260°C decomposed.

### EXAMPLE III

### 5'-DEOXY-5'-(3-AMINO-2-METHYLENEPROPYL)METHYLAMINOADENOSINE

### Step A: 1-PHTHALIMIDO-3-CHLORO-2-METHYLENEPROPANE

A mixture of 3-chloro-2-chloromethyl-1-propene (6.55 g, 50 mmol) and potassium phthalimide (5.6 g, 30 mmol) in anhydrous dimethylformamide (200 ml) was heated two days at 50°C. Then the mixture was concentrated *in vacuo* and, after usual work-up, the product was purified by flash chromatography on silica gel (ethyl acetate:hexane; 15:85) (4.2 g, 78%).

### Step B: 5'-DEOXY-5'-(3-PHTHALIMIDO-2-METHYLENEPROPYL)METHYLAMINO-2',3'-ISOPROPYLIDENEADENOSINE

A mixture of 1-phthalimido-3-chloro-2-methylenepropane (0.87 g, 5 mmol), potassium carbonate (0.7 g, 5 mmol), sodium iodide (0.08 g, 0.5 mmol) and 5'-deoxy-5'-methylamino-2',3'-isopropylideneadenosine (1.6 g, 5 mmol) in anhydrous acetonitrile (100 ml) was heated two days under reflux. Then the mixture was concentrated *in vacuo*, diluted with dichloromethane, filtered and the product was purified by flash chromatography on silica gel (diethyl amine:chloroform; 2:98) to give 2.85 g (78%) of the title compound.

### Step C: 5'-DEOXY-5'-(3-TERTIOBUTOXYCARBONYLAMINO-2-METHYLENEPROPYL)-METHYLAMINO-2',3'-ISOPROPYLIDENEADENOSINE

A mixture of 5'-deoxy-5'-(3-phthalimido-2-methylenepropyl)methylamino-2',3'-isopropylideneadenosine (2.3 g, 4.4 mmol), methyl hydrazine (1.5 ml, 30 mmol) in absolute ethanol (5 ml) was heated two days under reflux. Then the mixture was concentrated *in vacuo*, dissolved in chloroform (5 ml), the pH was adjusted around 9 with triethylamine and then a solution of ditertiobutyl dicarbonate (8.8g, 4.4 mmol) in chloroforn (5 ml) was added. The resulting mixture was heated overnight under reflux and, after usual work-up, the product was purified by flash chromatography on silica gel (diethylamine: chloroform; 2:98) to give 1.25 g (64%) of the title compound.

### Step D: 5'-DEOXY-5'-(3-AMINO-2-METHYLENEPROPYL)METHYLAMINOADENOSINE

A suspension of 5'-deoxy-5'-(3-tertiobutoxycarbonylamino-2-methylenepropyl)methylamino-2',3'-isopropylideneadenosine (0.65 g, 1.3 mmol) in 1N sulphuric acid (4 ml) was stirred two days at room temperature. Then the mixture was diluted with absolute ethanol (150 ml) and left at 0°C overnight. The precipitate was filtered off, dissolved in a minimum amount of water and diluted with absolute ethanol (150 ml). This procedure was repeated twice to afford the title compound as white crystals (0.55 g, mp: 230-240°C decomposed).

### EXAMPLE IV

### Preparation of CIS-5'-DEOXY-5'-(4-AMINO-2-FLUORO-2-BUTENYL)METHYLAMINOADENOSINE

### Step A: CIS-4-PHTHALIMIDO-2-FLUORO-1-TETRAHYDROPYRANYL-2-BUTENE

A mixture of cis-4-chloro-2-fluoro-1-tetrahydropyranyl-2-butene (6.3 g, 30 mmol) and potassium phthalimide (5.6 g, 30 mmol) in anhydrous dimethyl formamide (200 ml) was heated at 50°C for 24 h. Then the reaction mixture was concentrated *in vacuo*, dissolved in ethyl acetate, washed with brine and the pure title compound cis-4-phthalimido-2-fluoro-2-tetrahydropyranyl-2-butene (6 g, 70%) was obtained by flash chromatography on silica gel (ethyl acetate:hexane; 2:8).

### Step B: CIS-N-TERTIOBUTOXYCARBONYL-2-FLUORO-4-HYDROXY-2-BUTENYL-1-AMINE

A solution of cis-4-phthalimido-2-fluoro-2-tetrahydropyranyl-2-butene (5.7 g, 20 mmol) and hydrazine hydrate (1.1 ml, 22 mmol) in ethanol (30 ml) was heated under reflux for 12 h. Then the mixture was concentrated *in vacuo*, diluted with 1N HCl (20 ml) and heated under reflux for 2 h. Then the phthalhydrazide was filtered off and the filtrate was concentrated *in vacuo*. The residue was taken up in dichloromethane (150 ml), neutralized with triethylamine until pH 9, and a solution of ditertiobutyldicarbonate (5 g, 22 mmol) in dichloromethane (10 ml) was added. The mixture was heated under reflux overnight and, after usual work-up, the product was obtained by flash chromatography on silica gel (ethyl acetate:hexane; 25:75) (3 g, 75%).

### Step C: CIS-N-TERTIOBUTOXYCARBONYL-2-FLUORO-4-CHLORO-2-BUTENYL-1-AMINE

Mesylchloride (0.9 ml, 11 mmol) was added to a cold (0°C) solution of cis-N-tertiobutoxycarbonyl-2-fluoro-4-hydroxy-3-butenyl-1-amine (2.05 g, 10 mmol) and triethylamine (1.6 ml, 11 mmol) in anhydrous dichloromethane (40 ml). The mixture was stirred overnight and, after usual work-up, the title compound cis-N-tertiobutoxycarbonyl-2-fluoro-4-chloro-2-butenyl-1-amine was obtained by flash chromatography on silica gel (ethyl acetate:hexane; 15:85) (1.7 g, 75%).

### Step D: CIS-5'-DEOXY-5'-(4-TERTIOBUTOXYCARBONYLAMINO-2-FLUORO-2-BUTENYL)METHYLAMINO-2',3'-ISOPROPYLIDENEADENOSINE

A solution of 5'-deoxy-5'-methylamino-2',3'-isopropylideneadenosine (1.65 g, 5 mmol), cis-N-tertiobutoxycarbonyl-2-fluoro-4-chloro-2-butenyl-1-amine (1.2 g, 4 mmol), potassium carbonate (0.7 g, 4 mmol) and sodium iodide (0.07 g, 0.5 mmol) in anhydrous acetonitrile (30 ml) was heated under reflux overnight. The mixture was concentrated *in vacuo*, diluted with ethyl acetate, washed with brine and dried over MgSO₄. The product was purified by flash chromatography on silica gel (diethylamine: chloroform; 2:98) (1.7 g, 70%).

### Step E: CIS-5'-DEOXY-5'-(4-AMINO-2-FLUORO-2-BUTENYL)METHYLAMINOADENOSINE

A suspension of cis-5'-deoxy-5'-(4-tertiobutoxycarbonylamino-2-fluoro-2-butenyl)methylamino-2',3'-isopropylidene-adenosine in 1N sulphuric acid (5 ml) was stirred for 2 days at room temperature. Then the mixture was diluted with absolute ethanol (200 ml) and kept at 0°C overnight. The precipitate was collected, dissolved in a minimum of water, and reprecipitated with absolute ethanol (200 ml). This procedure was repeated twice to give the title compound cis-5'-deoxy-5'-(4-amino-2-fluoro-2-butenyl)methylaminoadenosine (1 g, 75%; mp: 250-260°C decomposed).

### EXAMPLE V

### Preparation of CIS-5'-DEOXY-5'-(4-CARBOXY-4-AMINO-2-BUTENYL)METHYLAMINOADENOSINE

### Step A: 2-AMINO-5-HYDROXY-3-PENTYNOIC ACID

A mixture of glyoxylic acid monohydrate (23 g, 250 mmol), propargyl alcohol (16.8 g, 300 mmol), copper (II) chloride (3.2 g, 25 mmol) and ammonium acetate (49 g, 600 mmol) in ethanol (100 ml) is heated under reflux for 6 h. Then the reaction mixture is concentrated *in vacuo*, diluted with water (50 ml), acidified to pH 5 with 1N HCl and washed twice with ether (100 ml). Then the aqueous solution is poured on an ion exchange resin column (DOWEX 50, H⁺). The column is eluted with 1 M ammonium hydroxide to give the title compound 2-amino-5-hydroxy-3-pentynoic acid.

### Step B: TERTIOBUTYL-2-AMINO-5-HYDROXY-3-PENTYNOATE

A suspension of 2-amino-5-hydroxy-3-pentynoic acid (12.5 g, 100 mmol) concentrated in sulphuric acid (2 ml) and isopropylene (50 ml) in a sealed Parr's flask is shaken 2 days at room temperature. The crude product, after evaporation of the excess of isopropylene, is used for the next step without further purification.

### Step C: TERTIOBUTYL-2-TERTIOBUTOXYCARBONYLAMINO-5-HYDROXY-3-PENTYNOATE

A solution of the crude tertiobutyl-2-amino-5-hydroxy-3-pentynoate (100 mmol), ditertiobutyldicarbonate (22 g, 100 mmol) and triethylamine (25 ml, 200 mmol) in chloroform is heated under reflux overnight. Then, after usual work-up, the product is purified by flash chromatography on silica gel (ethyl acetate:hexane; 20:80).

### Step D: CIS-TERTIOBUTYL-2-TERTIOBUTOXYCARBONYLAMINO-5-HYDROXY-3-PENTENOATE

A solution of tertiobutyl-2-tertiobutoxycarbonylamino-5-hydroxy-3-pentynoate (13.6 g, 50 mmol) in ethanol (200 ml) is hydrogenated in presence of Lindlar catalyst (0.6 g) at atmospheric pressure and room temperature. In 3 h one equivalent of hydrogen (1.1 liters) is taken up. Then the catalyst is removed by filtration and the mixture is concentrated *in vacuo* which will yield a clear oil. The title compound is obtained by flash chromatography on silica gel (ethyl acetate:hexane; 15:85).

### Step E: CIS-TERTIOBUTYL-2-TERTIOBUTOXYCARBONYLAMINO-5-CHLORO-3-PENTENOATE

Mesyl chloride (0.9 ml, 11 mmol) is added to a cold (0°C) solution of cis-tertiobutyl-2-tertiobutoxycarbonylamino-5-hydroxy-3-pentenoate (2.75 g, 10 mmol) and triethylamine (1.6 ml, 11 mmol) in anhydrous dichloromethane (50 ml). The mixture is stirred overnight and, after usual work-up, the title compound is purified by flash chromatography on silica gel (ethyl acetate:hexane; 20:80).

### Step F: CIS-5'-DEOXY-5'-(4-TERTIOBUTOXYCARBONYL-3-TERTIOBUTOXYCARBONYLAMINO-2-BUTENYL)METHYLAMINO-2',3'-ISOPROPYLIDENE-ADENOSINE

A solution of cis-tertiobutyl-2-tertiobutoxycarbonylamino-5-chloro-3-pentenoate (1.5 g, 5 mmol), 5'-deoxy-5'-methylamino-2',3'-isopropylideneadenosine (1.6 g, 5 mmol), potassium carbonate (0.7 g, 5 mmol) and sodium iodide (0.8 g, 5 mmol) in acetonitrile (30 ml) is heated under reflux overnight. After usual work-up, the product is purified by flash chromatography on silica gel (diethylamine:chloroform; 2:98).

### Step G: CIS-5'-DEOXY-5'-(4-CARBOXY-4-AMINO-2-BUTENYL)METHYLAMINOADENOSINE

A suspension of cis-5'-deoxy-5'-(4-tertiobutoxycarbonyl-3-tertiobutoxycarbonylamino-2-butenyl)methylamino-2'-,3'-isopropylideneadenosine (1.5 g, 3 mmol) in 1N sulphuric acid (5 ml) is stirred 2 days at room temperature. Then the mixture is diluted with ethanol (200 ml) and kept at 0°C overnight. The precipitate is collected, dissolved in a minimum amount of water, and reprecipitated with ethanol (200 ml). This procedure is repeated twice and will yield the title compound cis-5'-deoxy-5'-(4-carboxy-4-amino-2-butenyl)methylaminoadenosine.
(Usual work-up involves the extraction of the product from the aqueous phase by three extractions with the organic solvent (as in Step C, Example I) and the organic phase dried over magnesium sulfate, filtered off and concentrated *in vacuo*.)

The compounds of Formula I are inhibitors of decarboxylase enzymes which are involved in polyamine formation and therefore such compounds are useful as pharmacological agents. In particular, the compounds of Formula I are potent and irreversible inhibitors of S-adenosylmethionine decarboxylase (Ado Met DC) and therefore significantly interfere with the formation of spermine and spermidine and thus are useful adducts in the armentarium of researchers and clinicians in the study of polyamine formation and in the treatment of conditions and diseases related to the rapid proliferation of cell growth. Of particular importance is the use of the compounds of this invention in conjunction with known ornithine decarboxylase inhibitors, known antitumoral agents and with immunomodulators known for their use in diseases associated with the rapid proliferation of normal and transformed cells.

As is well known in the art, polyamines are associated with both normal and rapid proliferation of cells and, as is also well known, the levels of polyamines are high in embryonic systems, the testes and patients suffering from diseases associated with rapid proliferation of cell growth. It is also known that there is a correlation between the activity of the decarboxylase enzymes of ornithine, S-adenosylmethionine, arginine and lysine and polyamine formation. Thus with this interrelationship the compounds of this invention, by their unique ability to inhibit S-adenosylmethionine decarboxylase, are also useful to study the biochemical and pharmacological consequences of polyamine biosynthesis blockade in mammals, plants, bacteria and protozoa.

More specifically, some of the more promising end-use applications of the compounds of this invention are the following applications.

The use of an Ado Met DC inhibitor of this invention, alone but more effectively in conjunction with an ornithine decarboxylase (ODC) inhibitor in mammals as postcoital contraceptives, inducers of menstruation, and as first-trimester abortifacients is clear for such use does not surgically invade the cavity of the uterus, does not require hospitalization, and can be administered with the minimum of medical supervision.

As is well known the use of single agents in anti-neoplastic chemotherapy has been only marginally successful and limited to a very few malignancies. Thus treatment of such malignancies as leukemia (e.g., acute lymphocytic leukemia, Hodgkins' disease), melanoma and metastatic melanoma, multiple myeloma, bladder carcinoma, prostate carcinoma (as well as benign renal adenocarcinoma, gastric adenocarcinoma, prostatic hypertrophy), pancreatic adenocarcinoma, colon cancer, lung carcinoma, fibrosarcoma and mammary carcinoma will be enhanced by the use of the Ado Met DC inhibitors of this invention in conjunctive therapeutic regimens with prior art drug combinations. For example, the use of the instant Ado Met DC inhibitors, preferably with ODC inhibitors and/or with interferon will significantly facilitate a lowering of untoward side effects and increase survival time when used in conjunction with such well-known drug combinations as vincristine, amethopterin, mercaptopurine, prednisone (VAMP), cyclophosphamide, arabinosylcytosine, oncovin (COAP), mechlorethamine, procarbazine (MOPP), ABVD, CHOP, CAF, FAM and PVE (see review by Berger, N.A. (1986), J. Clin. Invest. (1131-1135). Additionally the use of the compounds of this invention improves the efficacy of the prevention of metastasis in cancers of the breast, colon and bladder with ODC inhibitors. Another specific clinical aspect of the compounds of this invention is the enhancement of the synergism between ODC inhibitors and α-interferon in the treatment of cancers, particularly metastatic malignant melanoma. Still another aspect of the enhancement of the unique interaction between ODC inhibitors and methylglyoxal bisguanylhydrazone (MGBG; methyl GAG), particularly in cases with recurrent primary brain tumors (e.g., astrocytomas). Still further, the compounds of this invention may be useful in the treatment of hyperproliferative disorders such as psoriasis.

In addition to the foregoing, the compounds of this invention may be used to treat diseases which are caused by infections with animal parasites, particularly parasitic protozoa and parasitic nematodes. In the treatment of diseases caused by these parasites, the compounds of this invention may be used alone, or in combination with ornithine decarboxylase inhibitors and/or in combination with other agents known to be useful in the treatment of such diseases. In some instances, such as in treating Chagas Disease, it is preferred to utilize arginine decarboxylase inhibitors in conjunction with the compounds of this invention. Of particular interest is the treatment of African trypanosomiasis, Chagas Disease and *Pneumocystis carinii* pneumonia (PCP) in patients suffering from AIDS (particularly in conjunction with an ODC inhibitor), cryptosporidiosis and malaria.

Still more specifically, the compounds of this invention are particularly useful in treating:
(1) diseases caused by *Onchocerca Volvulus*, a filarial nematode living in subcutaneous tissue causing skin lesions and eye lesions (river blindness). These diseases are commonly treated with diethylcarbamazine which kills the microfilariae, but not the adult worms;
(2) diseases caused by *Wuchereria bancrofti*, a thread-like nematode the adults of which live in thin lymphatic vessels and which cause lymphangitis, dermatitis and cellulitis. These diseases are commonly treated with diethylcarbamazine but this treatment is known to be inadequate;
(3) diseases caused by *Loa loa*, a filarial worm causing Loaiasis characterized by hot erythematous Calabar swelling on extremities and periorbital tissues which also have been treated with diethylcarbamazine;
(4) diseases caused by *Trichomonas vaginalis* a sexually transmitted flagellate protozoa causing trichomoniasis which is commonly treated with metronidazole;
(5) diseases caused by *Giardia lamblia*, a flagellated protozoan parasite causing giardiasis in domestic dogs and wild animals as well as man, for which the drugs of choice are quinacrine hydrochloride and metronidazole;
(6) diseases caused by *Toxoplasma gondii*, a protozoan parasite of the sub-class *coccidia* causing congenital toxoplasmosis which may be treated with pyrimethamine and sulfadiazine;
(7) diseases caused by the malarias of the genus *Plasmodium*, e.g., *Plasmodium falciparum*, *Plasmodium vivax*, *Plasmodium ovale* and *Plasmodium malariae* which are the causes of malaria; the usual treatment is with chloriquine, quinine sulfate, pyrimethanine and sulfadiazine. In the treatment of this disease it is better to utilize the compounds of this invention in conjunctive therapy with the foregoing or with ODC inhibitors;
(8) diseases caused by *Trypanosoma cruzi*, a protozoa causing Chagas Disease, a disease which has a history of being difficult to treat. In the treatment of this disease with the compounds of this invention it is recommended that they be used in conjunctive therapy with arginine and agmatine decarboxylase inhibitors;
(9) diseases caused by the African trypanosomes, *Trypanosoma brucei gambiense* and *Trypanosoma brucei rhodesiense*, two subspecies of hemoflagellates which are responsible for African Trypanosomiasis, a disease treated by suramin and more recently by eflornithine. Of particular use in the treatment of this disease are the specific compounds cis-5'-deoxy-5'(4-amino-2-butenyl)methylaminoadenosine and cis-5'-deoxy-5'(4-carboxy-4-amino-2-butenyl)methylaminoadenosine.
(10) diseases caused by *Leishmania tropica* and *Leishmania mexicana* which are responsible for cutaneous leishmaniasis, and visceral leishmaniasis (also called kala azar or black fever) which is caused by *Leishmania donovani*.

Furthermore, an Ado Met DC inhibitor of this invention may be used (alone or in combination with ODC inhibitors) as anti-infective agents being effective in the control of bacteria, fungi and viruses which are dependent upon polyamines for growth, for example *E.coli*, *Enterobacter*, *H.influenzae*, *Mycobacteria* species, *Staphylococcus aureus*, *Klebsiella*, viruses such as poxviruses, Herpes viruses, picornaviruses and influenza viruses. In the use of the compounds of this invention it is preferred to use such ODC inhibitors as α-difluoromethylornithine, α-monofluoromethylornithine, α-ethylornithine, (ε)-2-(fluoromethyl)dehydroornithine (and the methyl, ethyl and other esters thereof), and (2R,5R)-6-heptyne-2,5-diamine; said compounds and their uses being adequately described as to their preparation, their ODC inhibitory properties and to their end-use applications (see Inhibition of Polyamine Metabolism, (1987) edited by McCann, P.P., Pegg, A.E., and Sjoerdsma, A.).

The S-adenosylmethionine decarboxylase inhibitory properties of the compounds of Formula I may readily be determined by standard laboratory procedures well known in the art. For example, cis-5'-deoxy-5'-(4-amino-2-butenyl)methylaminoadenosine produces inactivation of rat liver Ado Met DC *in* *vitro* with a t½ = 16 minutes at 0.1 µM, a t½ = 1.6 minutes at 1 µM and a t½ = 0.8 minutes at 2 µM.

As pharmacologically useful agents the compounds of Formula I can be administered in various manners to the patient being treated to achieve the desired effect. The compounds can be administered alone or in combination with another according to standard techniques for conjunctive therapy, bearing in mind that the compounds of this invention preferably enhance established protocols when used to treat neoplasms. The compounds are preferably administered in the form of a pharmaceutical preparation. In general, the compounds may be administered orally, parenterally, for example, intravenously, intraperitoneally, or subcutaneously, infusionally or topically, as determined by factors well-known and appreciated by the skilled artisan. The amount of compound administered will vary over a wide range and can be any effective amount, depending on the patient to be treated, the condition being treated and the mode of administration. The effective amount of compound administered will vary from about 0.2 mg/kg to 200 mg/kg of body weight of the patient per treatment dose and preferably will be about 1 mg/kg to about 50 mg/kg of body weight of the patient per treatment dose.

The solid unit dosage forms can be of the conventional type. Thus, the solid form can be a capsule which can be of the ordinary gelatin type containing a novel compound of this invention and a carrier, for example, lubricant and inert fillers, such as lactose, sucrose and corn starch. In another embodiment, the novel compounds are tableted with conventional tablet bases such as lactose, sucrose or corn starch in combination with binders such as acacia, corn starch or gelatin, disintegrating agents such as corn starch, potato starch, or alginic acid, and a lubricant such as stearic acid, or magnesium stearate.

For parenteral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable or synthetic origin, for example, peanut oil, soybean oil, and mineral oil. In general, water, saline, aqueous dextrose, and related sugar solutions, ethanols and glycols such as propylene glycol or polyethylene glycol are preferred liquid carriers, particularly for injectable solutions.

The compounds can be administered in the form of a depot injection or implant preparation which may be formulated in such a manner as to permit a sustained release of the active ingredient. The active ingredients can be compressed into pellets or small cylinders and implanted subcutaneously or intramuscularly as depot injections or implants. Implants may employ inert materials such as biodegradable polymers or synthetic silicones, for example, Silastic, silicone rubber manufactured by the Dow-Corning Corporation.

As is true for most generic classes of compounds suitable for pharmaceutical end-use applications, certain sub-classes and certain specific compounds are preferred. For the compounds of this invention (I) those compounds wherein R is hydrogen or methyl are preferred and those compounds wherein Q represents formulae Ie, particularly the cis-configuration, and those of Ia. Preferred specific compounds are cis-5'-deoxy-(4-amino-2-butenyl)methylamino adenosine, cis-5'-deoxy-(4-amino-2-butenyl)amino adenosine and their 2-fluoro, 3-fluoro, and 2,3-difluoro analogs, and 5'-deoxy-5'-(3-amino-2-methylenepropyl)methylaminoadenosine,5'-deoxy-5'-(3-amino-2-methylenepropyl)aminoadenosine and the mono and difluoro analogs (X and/or Y of Ia are fluoro) thereof, and cis-5'-deoxy-5'-(4-amino-4-carboxy-2-butenyl)methylaminoadenosine and cis-5'-deoxy-5'-(4-amino-4-carboxy-2-butenyl)aminoadenosine.

## Claims (Claims for the following Contracting State(s): AT, BE, CH, DE, FR, GB, IT, LI, LU, NL, SE)

1. A compound of the formula 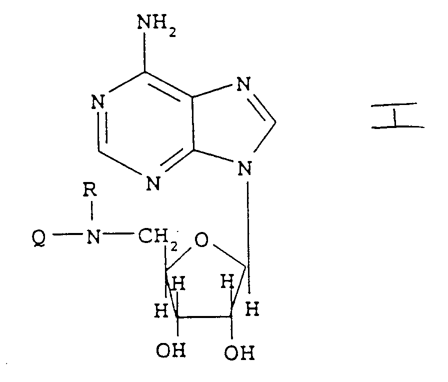 and the pharmaceutically acceptable salts thereof, wherein
R is hydrogen or a C₁₋₇ alkyl,
Q represents a moiety of one of the formulae Ia or Ie : 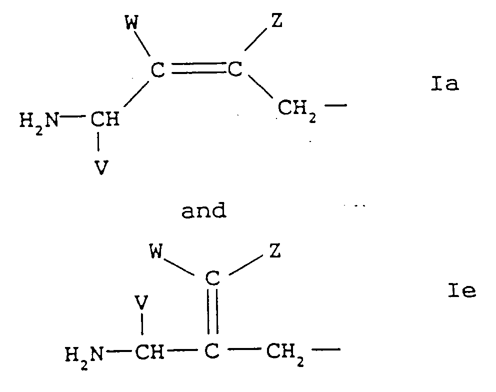 wherein
V is H or -COOH,
W is H, F, Cl or Br,
Z is H, F, Cl or Br.

2. A compound of claim 1 wherein R is methyl or H.

3. A compound of claim 1, said compound being cis-5'-deoxy-5'-(4-amino-2-butenyl)methylaminoadenosine.

4. A compound of claim 1, said compound being cis-5'-deoxy-5'-(4-amino-2-butenyl)aminoadenosine.

5. A compound of claim 1, said compound being cis-5'-deoxy-5'-(4-amino-4-carboxy-2-butenyl)methylaminoadenosine.

6. A compound of claim 1, said compound being cis-5'-deoxy-5'-(4-amino-4-carboxy-2-butenyl)aminoadenosine.

7. A compound of claim 1, said compound being 5'-deoxy-5'-(3-amino-2-methylenepropyl)methylaminoadenosine.

8. A compound of claim 1, said compound being 5'-deoxy-5'-(3-amino-2-methylenepropyl)aminoadenosine.

9. A compound of the formula 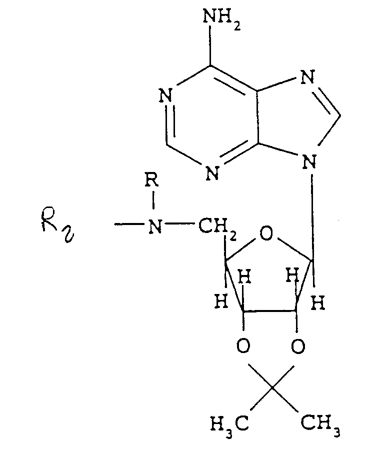 wherein R₂ is a moiety of one of the formulae : 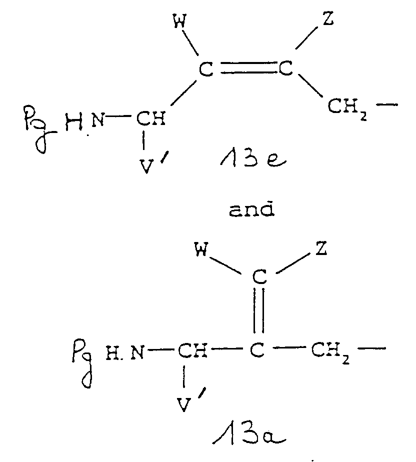 wherein
V' is H -COOH, or COOR₃ with R₃ being a reaction protection moiety,
W is H, F, Cl or Br,
Z is H, F, Cl or Br,
Pg is a N-protecting group, R is H or C₁₋₇ alkyl.

10. A process for preparing a compound of the formula 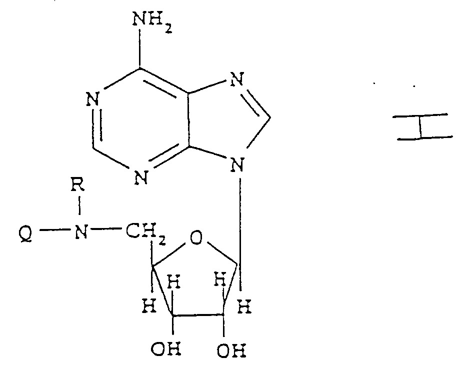 and the pharmaceutically acceptable salts thereof, wherein
R is hydrogen or a C₁₋₇ alkyl,
Q represents a moiety of one of the formulae Ia or Ie : 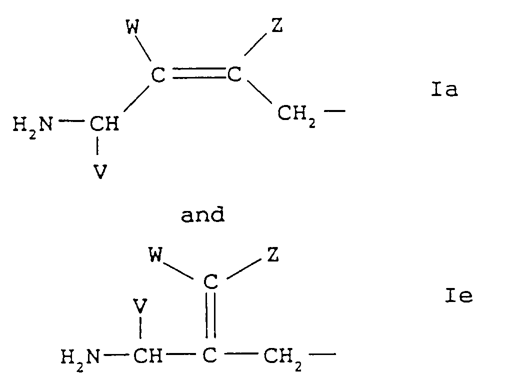 wherein
V is H or -COOH,
W is H, F, Cl or Br,
Z is H, F, Cl or Br.
which comprises reacting a compound of the formula 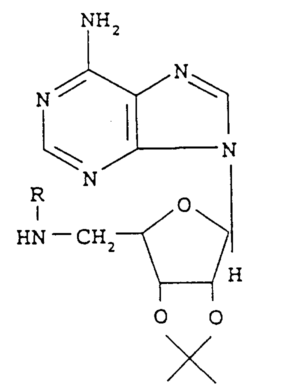 with a R₂X' reactant, R₂X' being a compound of the formula 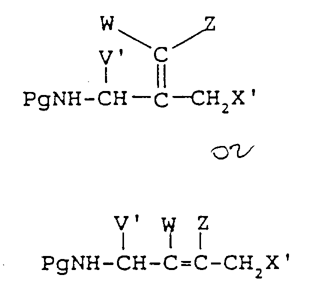 wherein Pg is an N-protecting moiety, V' is H, COOH or COOR₃ with R₃ being a reaction protection moiety, and X' is a triflate, chloro, bromo or iodo, said reaction being effected with equimolar quantities of the involved reactants in the presence of a base, preferably in a basic solvent, at temperatures of about 30°C to 80°C, followed by the removal of any protecting groups using standard techniques, and optionally converting the so-produced compounds to their pharmaceutically acceptable salts.

11. Pharmaceutical composition containing as active principle an effective amount of a compound according to any one of claims 1 to 9 in combination with a pharmaceutical carrier.

## Claims (Claims for the following Contracting State(s): ES)

1. Process for preparing a compound of the formula 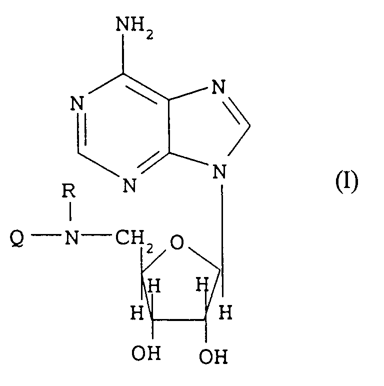 and the pharmaceutically acceptable salts thereof, wherein
R is hydrogen or a C₁₋₇ alkyl,
Q represents a moiety of one of the formulae Ia or Ie : 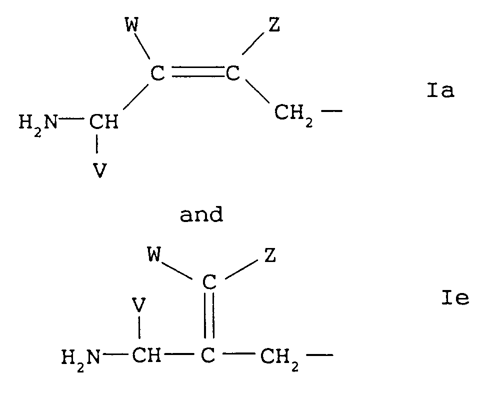 wherein
V is H or -COOH,
W is H, F, Cl or Br,
Z is H, F, Cl or Br,
which comprises reacting a compound of the formula 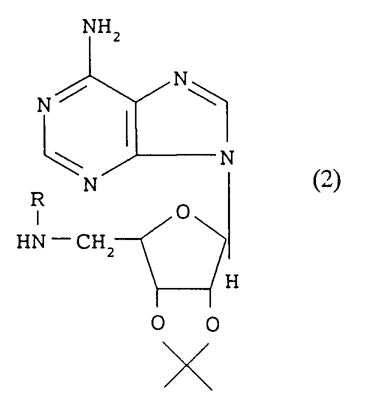 with a R₂X' reactant, R₂X' being a compound of the formula 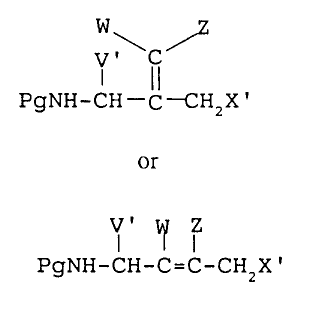 wherein Pg is an N-protecting moiety, V' is H, COOH or COOR₃ with R₃ being a reaction protection moiety, and X' is a triflate, chloro, bromo or iodo, said reaction being effected with equimolar quantities of the involved reactants in the presence of a base, preferably in a basic solvent, at temperatures of about 30°C to 80°C, followed by the removal of any protecting groups using standard techniques, and optionally converting the so-produced compounds to their pharmaceutically acceptable salts.

2. Process according to claim 1 in which a compound of formula (2) wherein R is methyl or H is used.

3. Process according to claim 1 which comprises reacting a compound of formula (2) wherein R is methyl with a R₂X' reactant of the formula 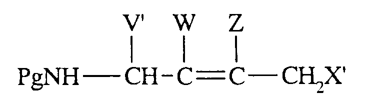 wherein V', W and Z are H,in order to obtain the cis-5'-deoxy-5'-(4-amino-2-butenyl)methylaminoadenosine.

4. Process according to claim 1, which comprises reacting a compound of formula (2) wherein R is H with a R₂X'reactant of the formula 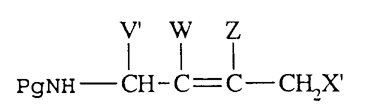 wherein V', W and Z are H, in order to obtain the cis-5'-deoxy-5'-(4-amino-2-butenyl)aminoadenosine.

5. Process according to claim 1 which comprises reacting a compound of formula (2) wherein R is methyl with a R₂X' reactant of the formula 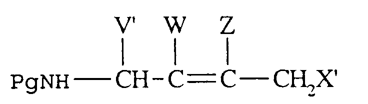 wherein V' is COOH, W and Z are both H, in order to obtain the cis-5'-deoxy-5'-(4-amino-4-carboxy-2-butenyl)methylaminoadenosine.

6. Process according to claim 1 which comprises reacting a compound of formula (2) wherein R is H with a R₂X' reactant of the formula 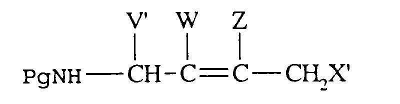 wherein V'is COOH, W and Z are both H, in order to obtain the cis-5'-deoxy-5'-(4-amino-4-carboxy-2-butenyl)aminoadenosine.

7. Process according to claim 1 which comprises reacting a compound of formula (2) wherein R is methyl with a R₂X' reactant of the formula 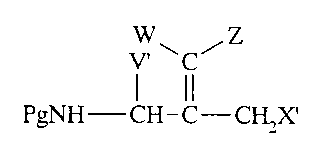 wherein V', W and Z are H, in order to obtain the 5'-deoxy-5'-(3-amino-2-methylenepropyl)methylaminoadenosine.

8. Process according to claim 1 which comprises reacting a compound of formula (2) wherein R is H with a R₂X' reactant of the formula 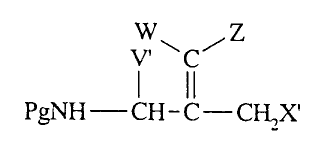 wherein V', W, Z are H, in order to obtain the 5'-deoxy-5'-(3-amino-2-methylenepropyl)aminoadenosine.

9. Process for preparing a compound of the formula : 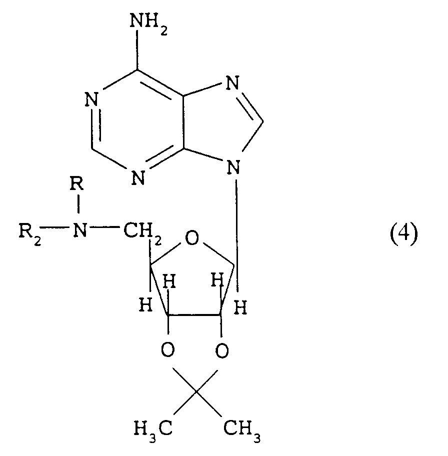 wherein R₂ is a moiety of one of the formulae : 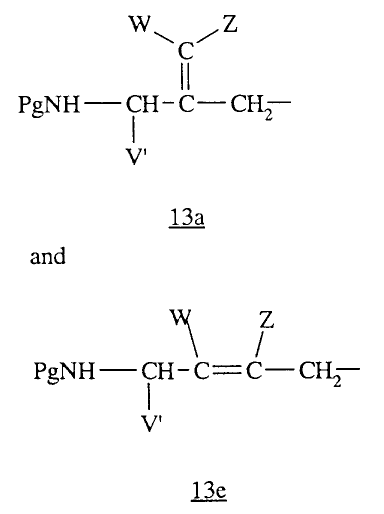 wherein,
V' is H, -COOH or -COOR₃ with R₃ being a reaction protection moiety ;
W is H, F, Cl or Br;
Z is H, F, Cl or Br;
Pg is a N-protecting group ;
R is hydrogen or a C₁₋₇ alkyl.
which comprises reacting a compound of the formula 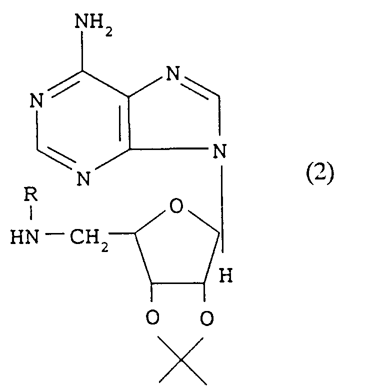 with a R₂X' reactant, R₂X' being a compound of the formula 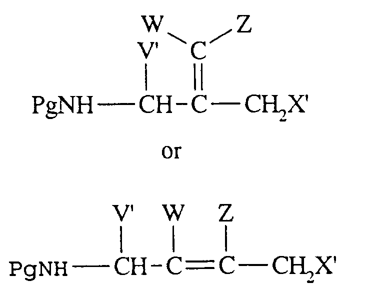 wherein Pg is an N-protecting moiety, V' is H, COOH or COOR₃ with R₃ being a reaction protection moiety, and X' is a triflate, chloro, bromo or iodo, said reaction being effected with equimolar quantities of the involved reactants in the presence of a base, preferably in a basic solvent, at temperatures of about 30° C to 80°C.

10. The use of a compound according to anyone of claims 1 to 9 for the preparation of pharmaceutical compositions.

## Claims (Claims for the following Contracting State(s): GR)

1. A compound of the formula 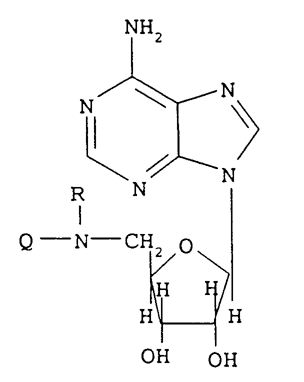 and the pharmaceutically acceptable salts thereof, wherein
R is hydrogen or a C₁₋₇ alkyl,
Q represents a moiety of one of the formulae Ia or Ie : 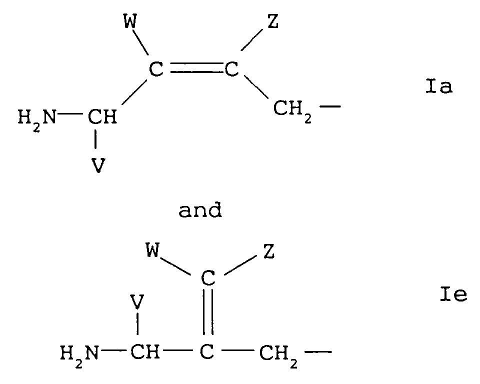 wherein
V is H or -COOH,
W is H, F, Cl or Br,
Z is H, F, Cl or Br.

2. A compound of claim 1 wherein R is methyl or H.

3. A compound of claim 1, said compound being cis-5'-deoxy-5'-(4-amino-2-butenyl)methylaminoadenosine.

4. A compound of claim 1, said compound being cis-5'-deoxy-5'-(4-amino-2-butenyl)aminoadenosine.

5. A compound of claim 1, said compound being cis-5'-deoxy-5'-(4-amino-4-carboxy-2-butenyl)methylaminoadenosine.

6. A compound of claim 1, said compound being cis-5'-deoxy-5'-(4-amino-4-carboxy-2-butenyl)aminoadenosine.

7. A compound of claim 1, said compound being 5'-deoxy-5'-(3-amino-2-methylenepropyl)methylaminoadenosine.

8. A compound of claim 1, said compound being 5'-deoxy-5'-(3-amino-2-methylenepropyl)aminoadenosine.

9. A compound of the formula 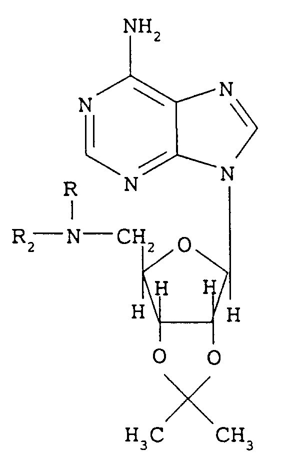 wherein R₂ is a moiety of one of the formulae : 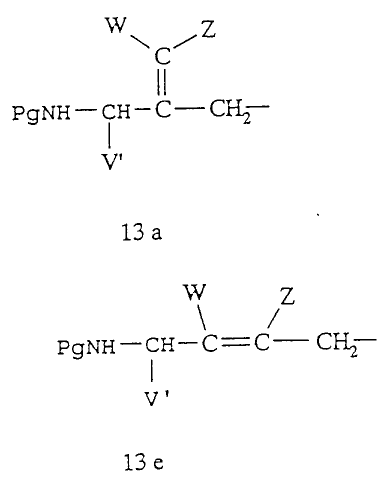 wherein
V' is H, -COOH or -COOR₃ with R₃ being a reaction protection moiety,
W is H, F, Cl or Br,
Z is H, F, Cl or Br,
Pg is a N-protecting group,
R is Hydrogen or a C₁-₇ alkyl.

10. A process for preparing a compound of the formula 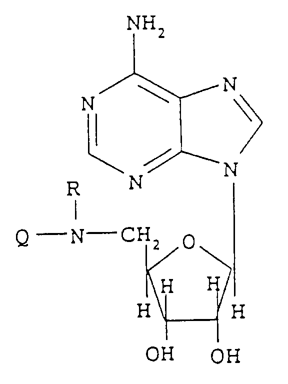 and the pharmaceutically acceptable salts thereof, wherein
R is hydrogen or a C₁₋₇ alkyl,
Q represents a moiety of one of the formulae Ia or Ie : 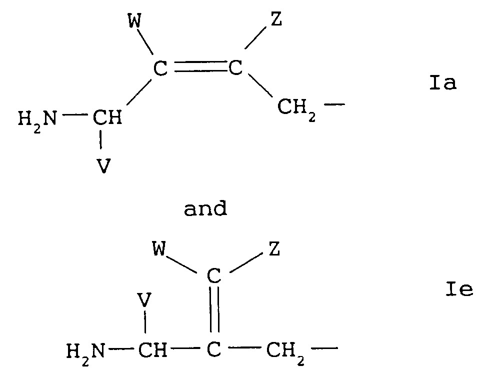 wherein
V is H or -COOH,
W is H, F, Cl or Br,
Z is H, F, Cl or Br.
which comprises reacting a compound of the formula 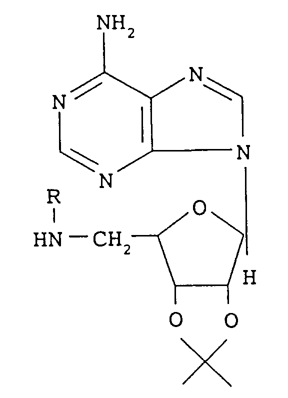 with a R₂X' reactant, R₂X' being a compound of the formula 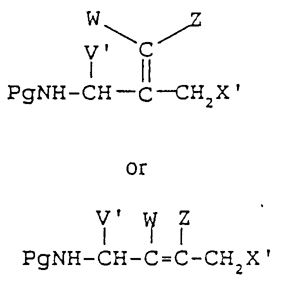 wherein Pg is an N-protecting moiety, V' is H, COOH or COOR₃ with R₃ being a reaction protection moiety, and X' is a triflate, chloro, bromo or iodo, said reaction being effected with equimolar quantities of the involved reactants in the presence of a base, preferably in a basic solvent, at temperatures of about 30°C to 80°C, followed by the removal of any protecting groups using standard techniques, and optionally converting the so-produced compounds to their pharmaceutically acceptable salts.

11. The use of a compound according to anyone of claims 1 to 9 for the preparation of pharmaceutical compositions.

## Patentansprüche (Patentansprüche für folgende(n) Vertragsstaat(en): AT, BE, CH, DE, FR, GB, IT, LI, LU, NL, SE)

1. Verbindung der Formel 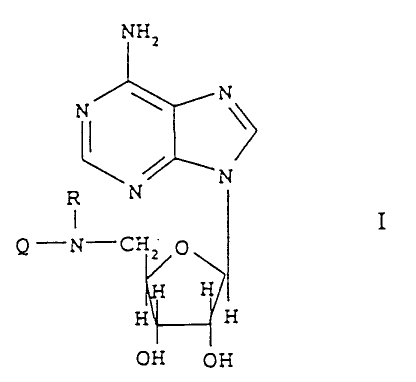 und die pharmazeutisch verträglichen Salze davon, wobei
R ein Wasserstoffatom oder ein C₁₋₇ Alkylrest ist,
Q eine Gruppe mit einer der Formeln Ia oder Ie darstellt: 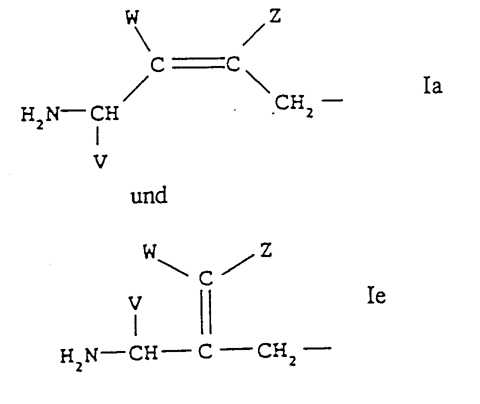 wobei
V H oder -COOH ist,
W H, F, Cl oder Br ist,
Z H, F, Cl oder Br ist.

2. Verbindung nach Anspruch 1, wobei R eine Methylgruppe oder H ist.

3. Verbindung nach Anspruch 1, wobei die Verbindung cis-5'-Desoxy-5'-(4-amino-2-butenyl)methylaminoadenosin ist.

4. Verbindung nach Anspruch 1, wobei die Verbindung cis-5'-Desoxy-5'-(4-amino-2-butenyl)aminoadenosin ist.

5. Verbindung nach Anspruch 1, wobei die Verbindung cis-5'-Desoxy-5'-(4-amino-4-carboxy-2-butenyl)methylaminoadenosin ist.

6. Verbindung nach Anspruch 1, wobei die Verbindung cis-5'-Desoxy-5'-(4-amino-4-carboxy-2-butenyl)aminoadenosin ist.

7. Verbindung nach Anspruch 1, wobei die Verbindung 5'-Desoxy-5'-(3-amino-2-methylenpropyl)methylaminoadenosin ist.

8. Verbindung nach Anspruch 1, wobei die Verbindung 5'-Desoxy-5'-(3-amino-2-methylenpropyl)aminoadenosin ist.

9. Verbindung der Formel 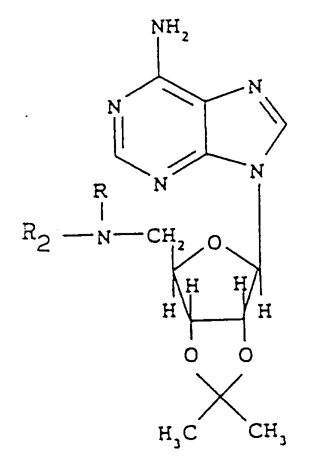 in der R₂ eine Gruppe mit einer der Formeln 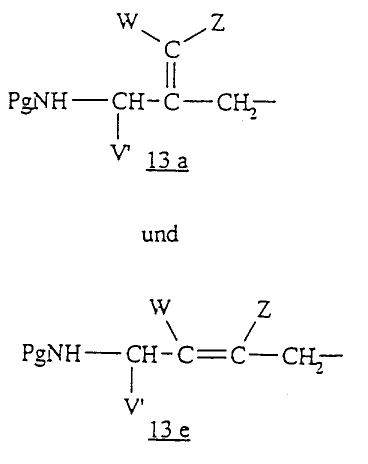 ist, wobei
V' H, -COOH, oder COOR₃ ist, wobei R₃ eine Reaktionsschutzgruppe ist;
W H, F, Cl oder Br ist;
Z H, F, Cl oder Br ist;
Pg eine N-Schutzgruppe ist;
R ein Wasserstoffatom oder ein C₁₋₇-Alkylrest ist.

10. Verfahren zur Herstellung einer Verbindung der Formel 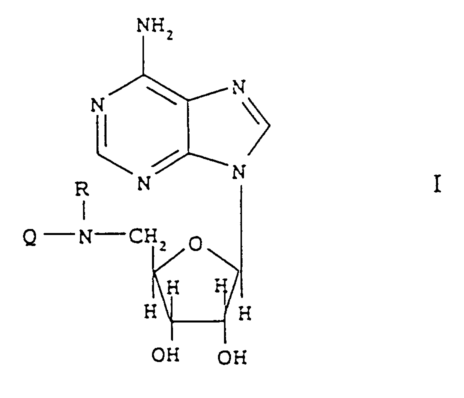 und der pharmazeutisch verträglichen Salze davon, wobei
R ein Wasserstoffatom oder ein C₁₋₇ Alkylrest ist,
Q eine Gruppe mit einer der Formeln Ia oder Ie darstellt: 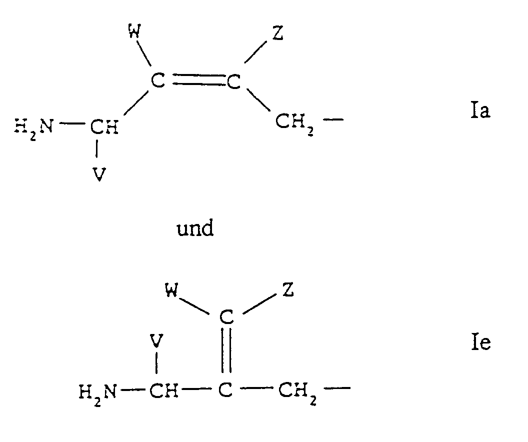 wobei
V H oder -COOH ist,
W H,F, Cl oder Br ist,
Z H, F, Cl oder Br ist,
das die Umsetzung einer Verbindung der Formel 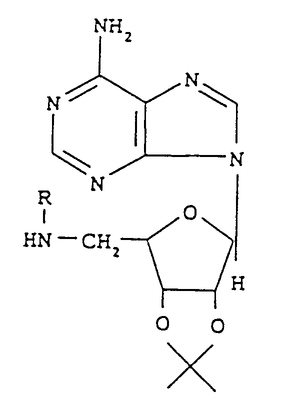 mit einem R₂X'-Reaktionsteilnehmer umfasst, wobei R₂X' eine Verbindung der Formel 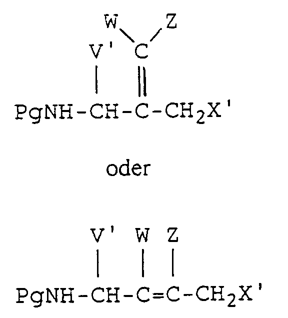 ist, wobei Pg eine N-Schutzgruppe ist, V' H, COOH oder COOR₃ ist, wobei R₃ eine Reaktions-Schutzgruppe ist, und X' eine Triflat-Gruppe, ein Chlor-, Brom- oder Iodatom ist, und wobei die Umsetzung mit äquimolaren Mengen der betreffenden Reaktionsteilnehmer in Gegenwart einer Base, vorzugsweise in einem basischen Lösungsmittel, bei Temperaturen von etwa 30°C bis 80°C durchgeführt wird, mit anschließender Entfernung aller Schutzgruppen nach Standardmethoden, und gegebenenfalls Umwandlung der so hergestellten Verbindungen in ihre pharmazeutisch verträglichen Salze.

11. Arzneimittel, als Wirkstoff enthaltend eine wirksame Menge einer Verbindung nach einem der Ansprüche 1 bis 9 in Verbindung mit einer pharmazeutischen Trägersubstanz.

## Patentansprüche (Patentansprüche für folgende(n) Vertragsstaat(en): ES)

1. Verfahren zur Herstellung einer Verbindung der Formel 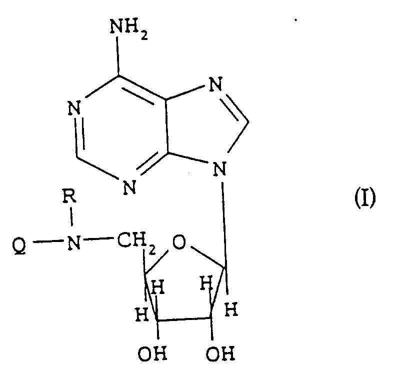 und der pharmazeutisch verträglichen Salze davon, wobei
R ein Wasserstoffatom oder ein C₁₋₇ Alkylrest ist,
Q eine Gruppe einer der Formeln Ia oder Ie darstellt: 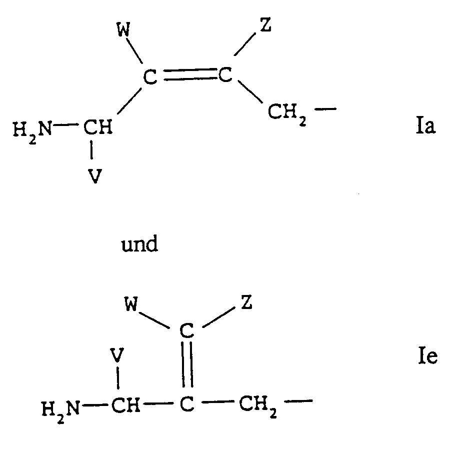 wobei
V H oder -COOH ist,
W H, F, Cl oder Br ist,
Z H, F, Cl oder Br ist,
das die Umsetzung einer Verbindung der Formel 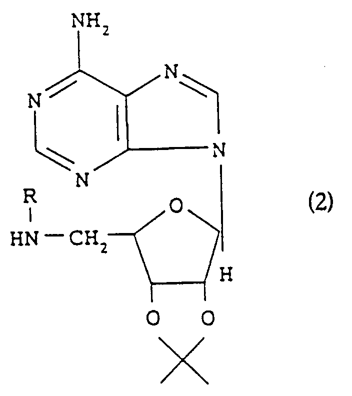 mit einem R₂X'-Reaktionsteilnehmer umfasst, wobei R₂X' eine Verbindung der Formel 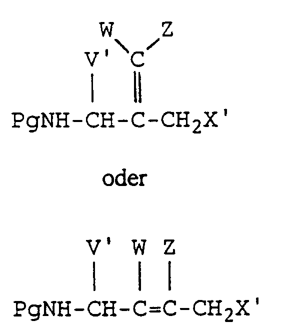 ist, in der Pg eine N-Schutzgruppe ist, V' H, COOH oder COOR₃ ist, wobei R₃ eine Reaktions-Schutzgruppe ist, und X' eine Triflat-Gruppe, ein Chlor-, Brom- oder Iodatom ist, und wobei die Umsetzung mit äquimolaren Mengen der betreffenden Reaktionsteilnehmer in Gegenwart einer Base, vorzugsweise in einem basischen Lösungsmittel, bei Temperaturen von etwa 30°C bis 80°C durchgeführt wird, mit anschließender Entfernung aller Schutzgruppen nach Standardmethoden, und gegebenenfalls Umwandlung der so hergestellten Verbindungen in ihre pharmazeutisch verträglichen Salze.

2. Verfahren nach Anspruch 1, bei dem eine Verbindung der Formel (2), in der R eine Methylgruppe oder ein Wasserstoffatom ist, verwendet wird.

3. Verfahren nach Anspruch 1, das die Umsetzung einer Verbindung der Formel (2), in der R eine Methylgruppe ist, mit einem Reaktionsteilnehmer R₂X' der Formel 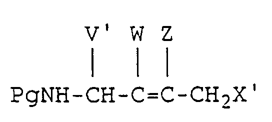 umfasst, in der R eine Methylgruppe ist, um cis-5'-Desoxy-5'-(4-amino-2-butenyl)methylaminoadenosin zu erhalten.

4. Verfahren nach Anspruch 1, das die Umsetzung einer Verbindung der Formel (2), in der R ein Wasserstoffatom ist, mit einem Reaktionsteilnehmer R₂X' der Formel 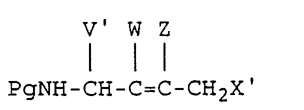 umfasst, in der V', W und Z H sind, um cis-5'-Desoxy-5'-(4-amino-2-butenyl)aminoadenosin zu erhalten.

5. Verfahren nach Anspruch 1, das die Umsetzung einer Verbindung der Formel (2), in der R eine Methylgruppe ist, mit einem Reaktionsteilnehmer R₂X' der Formel 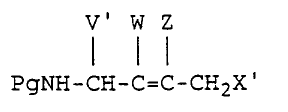 umfasst, in der V' COOH ist, W und Z beide H sind, um cis-5'-Desoxy-5'-(4-amino-4-carboxy-2-butenyl)methylaminoadenosin zu erhalten.

6. Verfahren nach Anspruch 1, das die Umsetzung einer Verbindung der Formel (2), in der R ein Wasserstoffatom ist, mit einem Reaktionsteilnehmer R₂X' der Formel 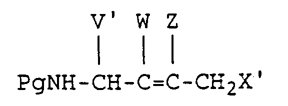 umfasst, in der V' COOH ist, W und Z beide H sind, um cis-5'-Desoxy-5'-(4-amino-4-carboxy-2-butenyl)aminoadenosin zu erhalten.

7. Verfahren nach Anspruch 1, das die Umsetzung einer Verbindung der Formel (2), in der R eine Methylgruppe ist, mit einem Reaktionsteilnehmer R₂X' der Formel 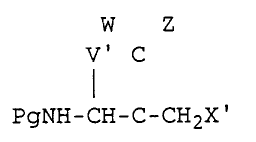 umfasst, in der V', W und Z H sind, um 5'-Desoxy-5'-(3amino-2-methylenpropyl)methylaminoadenosin zu erhalten.

8. Verfahren nach Anspruch 1, das die Umsetzung einer Verbindung der Formel (2), bei der R ein Wasserstoffatom ist, mit einem Reaktionsteilnehmer R₂X' der Formel 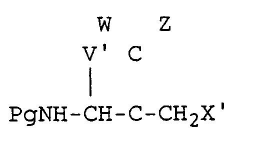 umfasst, in der V', W und Z H sind, um 5'-Desoxy-5'-(3-amino-2-methylenpropyl)aminoadenosin zu erhalten.

9. Verfahren zur Herstellung einer Verbindung der Formel: 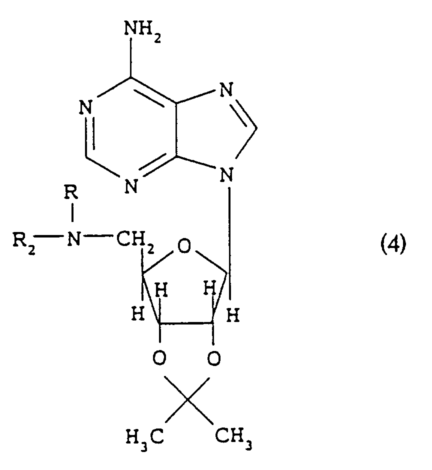 in der R₂ eine Gruppe einer der Formeln 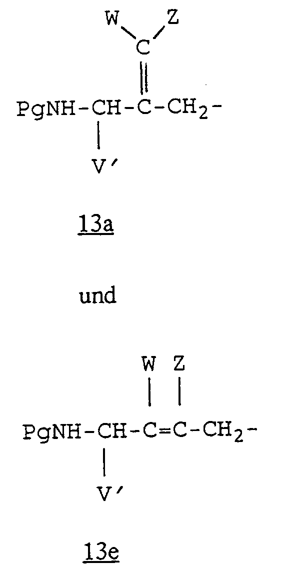 ist,
wobei
V' H, -COOH, oder COOR₃ ist, wobei R₃ eine Reaktionsschutzgruppe ist;
W H, F, Cl oder Br ist;
Z H, F, Cl oder Br ist;
Pg eine N-Schutzgruppe ist;
R ein Wasserstoffatom oder ein C₁₋₇-Alkylrest ist,
das die Umsetzung einer Verbindung der Formel 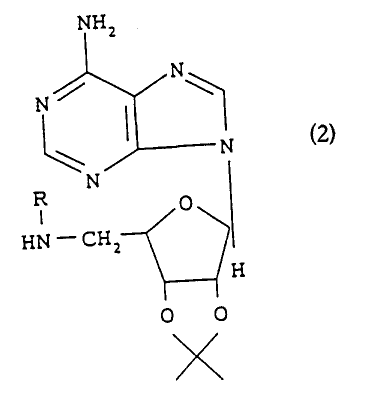 mit einem Reaktionsteilnehmer R₂X' umfasst, wobei R₂X' eine Verbindung der Formel 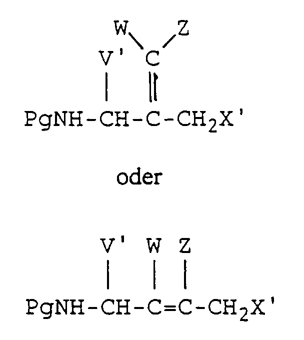 ist, in der Pg eine N-Schutzgruppe ist, V' H, COOH oder COOR₃ ist, wobei R₃ eine Reaktions-Schutzgruppe ist, und X' eine Triflat-Gruppe, ein Chlor-, Brom- oder Iodatom ist, und wobei die Umsetzung mit äquimolaren Mengen der betreffenden Reaktionsteilnehmer in Gegenwart einer Base, vorzugsweise in einem basischen Lösungsmittel, bei Temperaturen von etwa 30°C bis 80°C durchgeführt wird.

10. Verwendung einer Verbindung nach einem der Ansprüche 1 bis 9 zur Herstellung von Arzneimitteln.

## Patentansprüche (Patentansprüche für folgende(n) Vertragsstaat(en): GR)

1. Verbindung der Formel 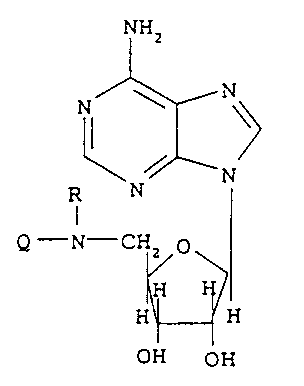 und die pharmazeutisch verträglichen Salze davon, wobei
R ein Wasserstoffatom oder ein C₁₋₇-Alkylrest ist
Q eine Gruppe einer der Formeln Ia oder Ie darstellt 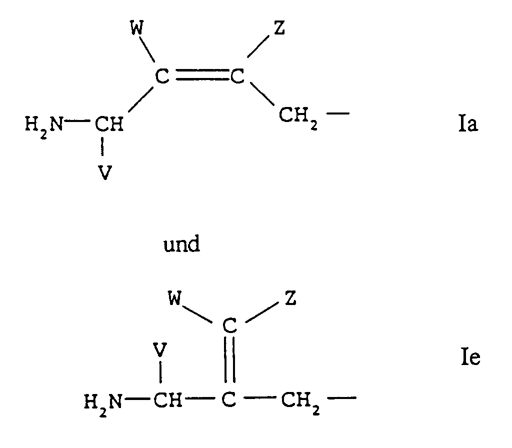 wobei
V H oder -COOH ist,
W H, F, Cl oder Br ist,
Z H, F, Cl oder Br ist.

2. Verbindung nach Anspruch 1, wobei R eine Methylgruppe oder H ist.

3. Verbindung nach Anspruch 1, wobei die Verbindung cis-5'-Desoxy-5'-(4-amino-2-butenyl)methylaminoadenosin ist.

4. Verbindung nach Anspruch 1, wobei die Verbindung cis-5'-Desoxy-5'-(4-amino-2-butenyl)aminoadenosin ist.

5. Verbindung nach Anspruch 1, wobei die Verbindung cis-5'-Desoxy-5'-(4-amino-4-carboxy-2-butenyl)methylaminoadenosin ist.

6. Verbindung nach Anspruch 1, wobei die Verbindung cis-5'-Desoxy-5'-(4-amino-4-carboxy-2-butenyl)aminoadenosin ist.

7. Verbindung nach Anspruch 1, wobei die Verbindung 5'-Desoxy-5'-(3-amino-2-methylenpropyl)methylaminoadenosin ist.

8. Verbindung nach Anspruch 1, wobei die Verbindung 5'-Desoxy-5'-(3-amino-2-methylenpropyl)aminoadenosin ist.

9. Verbindung der Formel 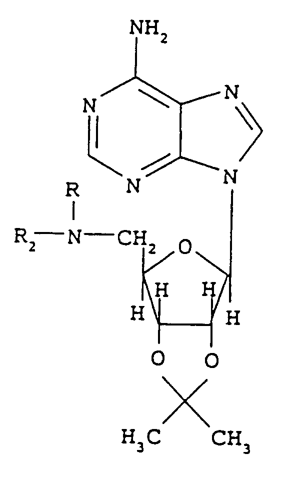 in der R₂ eine Gruppe mit einer der Formeln 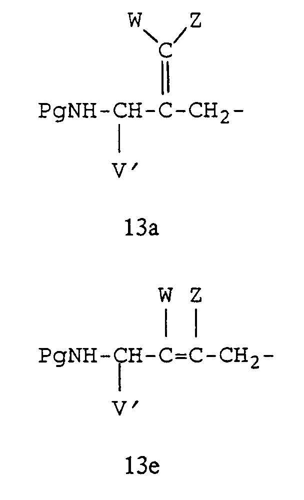 ist, wobei
V' H, -COOH, oder COOR₃ ist, wobei R₃ eine Reaktionsschutzgruppe ist;
W H, F, Cl oder Br ist;
Z H, F, Cl oder Br ist;
Pg eine N-Schutzgruppe ist;
R ein Wasserstoffatom oder ein C₁₋₇-Alkylrest ist.

10. Verfahren zur Herstellung einer Verbindung der Formel 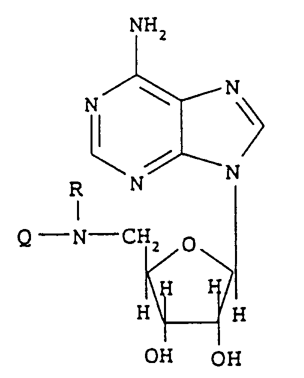 und der pharmazeutisch verträglichen Salze davon, wobei
R ein Wasserstoffatom oder ein C₁₋₇ Alkylrest ist,
Q eine Gruppe mit einer der Formeln Ia oder Ie darstellt: 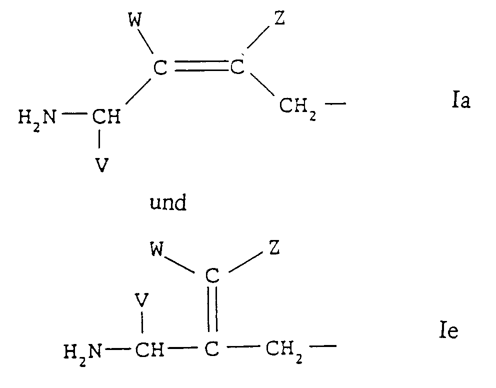 wobei
V H oder -COOH ist,
W H,F, Cl oder Br ist,
Z H, F, Cl oder Br ist,
das die Umsetzung einer Verbindung der Formel 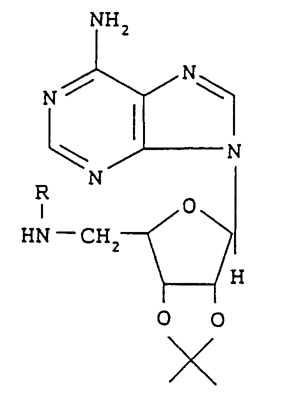 mit einem Reaktionseilnehmer R₂X' umfasst, wobei R₂X' eine Verbindung der Formel 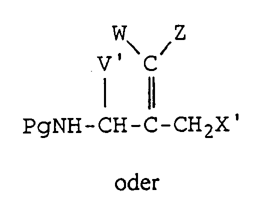 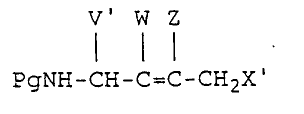 ist, wobei Pg eine N-Schutzgruppe ist, V' H, COOH oder COOR₃ ist, wobei R₃ eine Reaktions-Schutzgruppe ist, und X' eine Triflat-Gruppe, ein Chlor-, Brom- oder Iodatom ist, und wobei die Umsetzung mit äquimolaren Mengen der betreffenden Reaktionsteilnehmer in Gegenwart einer Base, vorzugsweise in einem basischen Lösungsmittel, bei Temperaturen von etwa 30°C bis 80°C durchgeführt wird, mit anschließender Entfernung aller Schutzgruppen nach Standardmethoden, und gegebenenfalls Umwandlung der so hergestellten Verbindungen in ihre pharmazeutisch verträglichen Salze.

11. Verwendung einer Verbindung nach einem der Ansprücche 1 bis 9 zur Herstellung von Arzneimitteln.

## Revendications (Revendications pour l'(les) Etat(s) contractant(s) suivant(s): AT, BE, CH, DE, FR, GB, IT, LI, LU, NL, SE)

1. Composé de formule : 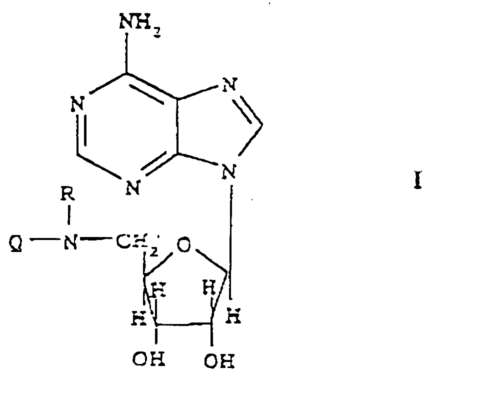 et ses sels pharmaceutiquement acceptables, où
R est l'hydrogène ou un alkyle en C₁-C₇,
Q représente une entité selon l'une des formules Ia ou Ie : 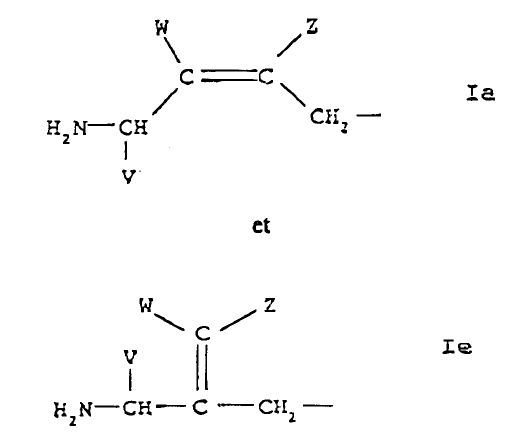 où
V est H ou -COOH,
W est H, F, Cl ou Br,
Z est H, F, Cl ou Br.

2. Composé selon la revendication 1, dans lequel R est méthyle ou H.

3. Composé selon la revendication 1, ledit composé étant la cis-5'-désoxy-5'-(4-amino-2-butényl)méthylaminoadénosine.

4. Composé selon la revendication 1, ledit composé étant la cis-5'-désoxy-5'-(4-amino-2-butényl)aminoadénosine.

5. Composé selon la revendication 1, ledit composé étant la cis-5'-désoxy-5'-(4-amino-4-carboxy-2-butényl)méthylaminoadénosine.

6. Composé selon la revendication 1, ledit composé étant la cis-5'-désoxy-5'-(4-amino-4-carboxy-2-butényl)amimoadénosine.

7. Composé selon la revendication 1, ledit composé étant la 5'-désoxy-5'-(3-amino-2-méthylènepropyl)méthylaminoadénosine.

8. Composé selon la revendication 1, ledit composé étant la 5'-désoxy-5'-(3-amino-2-méthylènepropyl)aminoadénosine.

9. Composé de formule : 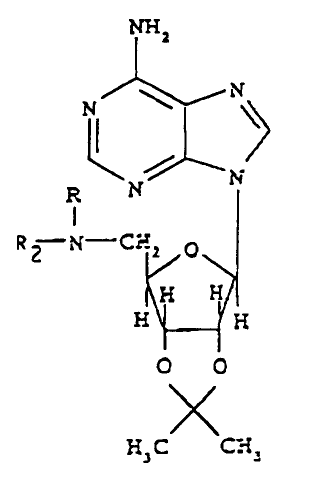 où R₂ est une entité selon l'une des formules : 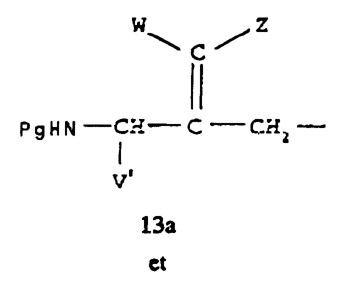 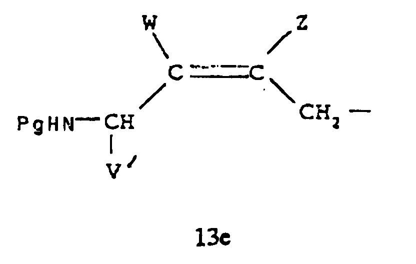 où
V' est H, COOH ou COOR₃, R₃ étant une entité de protection réactionelle ;
W est H, F, Cl ou Br,
Z est H, F, Cl ou Br,
Pg est un groupe N-protecteur,
R est H ou alkyle en C₁-C₇.

10. Procédé de préparation d'un composé de formule : 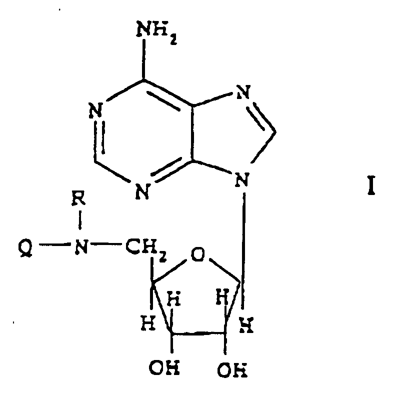 et de ses sels pharmaceutiquement acceptables, où
R est l'hydrogène ou un alkyle en C₁-C₇,
Q représente une entité selon l'une des formules Ia ou Ie : 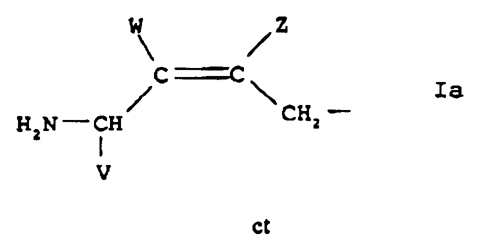 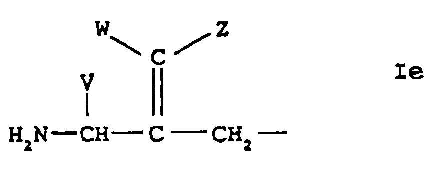 où
V est H ou COOH,
W est H, F, Cl ou Br,
Z est H, F, Cl ou Br,
qui comprend la réaction d'un composé de formule : 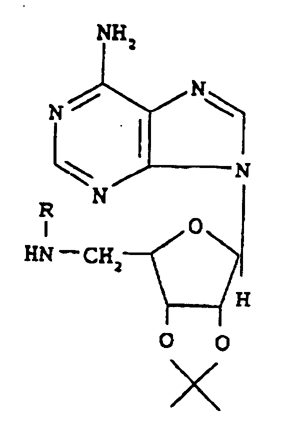 avec un corps réagissant R₂X', R₂X' étant un composé de formule : 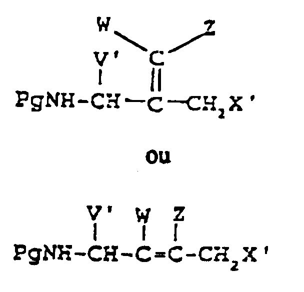 où Pg est une entité N-protectrice, V' est H, COOH ou COOR₃, R₃ étant une entité de protection réactionelle, et X' est un triflate, chloro, bromo ou iodo, ladite réaction étant conduite avec des quantités équimolaires des corps réagissants impliqués, en présence d'une base, de préférence dans un solvant basique, à des températures d'environ 30°C à 80°C, puis l'élimination de tous les groupes protecteurs à l'aide de techniques standards, et éventuellement la transformation des composés ainsi produits en leurs sels pharmaceutiquement acceptables.

11. Composition pharmaceutique contenant comme principe actif une quantité efficace d'un composé selon l'une quelconque des revendications 1 à 9 en combinaison avec un véhicule pharmaceutique.

## Revendications (Revendications pour l'(les) Etat(s) contractant(s) suivant(s): ES)

1. Procédé de préparation d'un composé de formule : 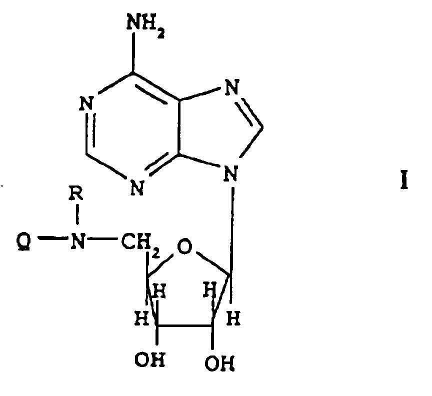 et de ses sels pharmaceutiquement acceptables, où
R est l'hydrogène ou un alkyle en C₁-C₇,
Q représente une entité selon l'une des formules Ia ou Ie : 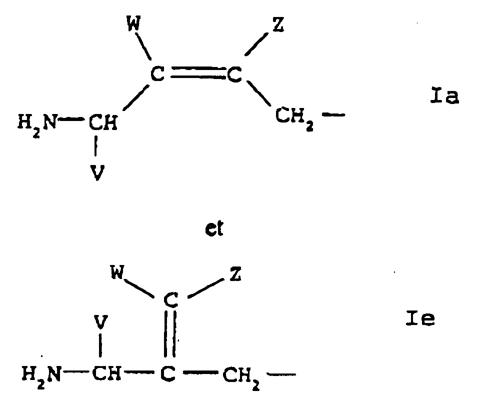 où
V est H ou -COOH,
W est H, F, Cl ou Br,
Z est H, F, Cl ou Br,
qui comprend la réaction d'un composé de formule : 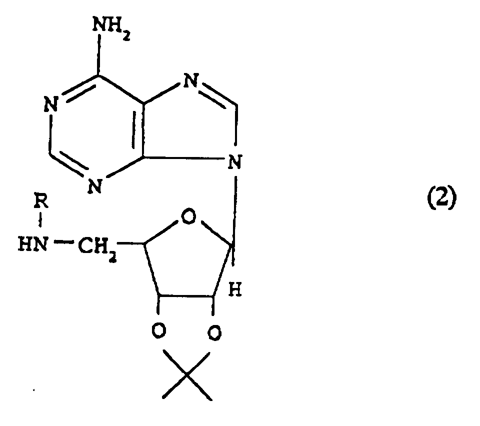 avec un corps réagissant R₂X', R₂X' étant un composé de formule : 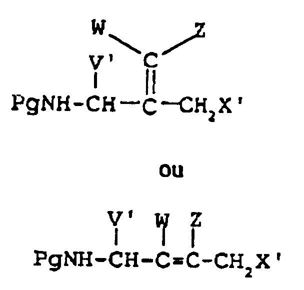 où Pg est une entité N-protectrice, V' est H, COOH ou COOR₃, R₃ étant une entité de protection réactionnelle, et X' est un triflate, chloro, bromo ou iodo, ladite réaction étant conduite avec des quantités équimolaires des réagissants impliqués, en présence d'une base, de préférence dans un solvant basique, à des températures d'environ 30°C à 80°C, puis l'élimination de tous les groupes protecteurs à l'aide de techniques standards, et éventuellement la transformation des composés ainsi produits en leurs sels pharmaceutiquement acceptables.

2. Procédé selon la revendication 1 dans lequel on utilise un composé de formule (2) où R est méthyle ou H.

3. Procédé selon la revendication 1 qui comprend la réaction d'un composé de formule (2) où R est méthyle avec un corps réagissant R₂X' de formule: 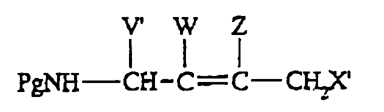 dans laquelle V', W et Z sont H, pour obtenir la cis-5'-désoxy-5'-(4-amino-2-butényl)méthylaminoadénosine.

4. Procédé selon la revendication 1 qui comprend la réaction d'un composé de formule (2) où R est H avec un corps réagissant R₂X' de formule : 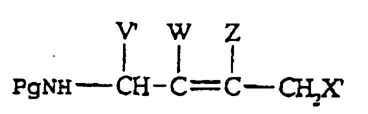 dans laquelle V', W et Z sont H, pour obtenir la cis-5'-désoxy-5'-(4-amino-2-butényl)aminoadénosine.

5. Procédé selon la revendication 1 qui comprend la réaction d'un composé de formule (2) où R est méthyle avec un corps réagissant R₂X' de formule : 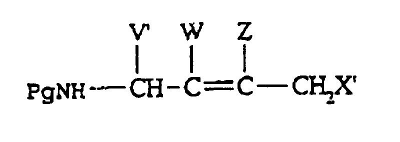 dans laquelle V' est COOH, W et Z sont tous les deux H, pour obtenir la cis-5'-désoxy-5'-(4-amino-4-carboxy-2-butényl)méthylaminoadénosine.

6. Procédé selon la revendication 1 qui comprend la réaction d'un composé de formule (2) où R est H avec un corps réagissant R₂X' de formule : 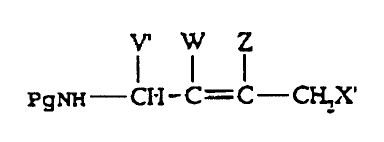 dans laquelle V' est COOH, W et Z sont tous les deux H, pour obtenir la cis-5'-désoxy-5'-(4-amino-4-carboxy-2-butényl)aminoadénosine.

7. Procédé selon la revendication 1 qui comprend la réaction d'un composé de formule (2) où R est méthyle avec un corps réagissant R₂X' de formule : 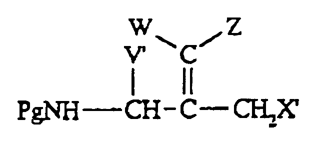 dans laquelle V', W et Z sont H, pour obtenir la 5'-désoxy-5'-(3-amino-2-méthylènepropyl)méthylaminoadénosine.

8. Procédé selon la revendication 1 qui comprend la réaction d'un composé de formule (2) où R est H avec un corps réagissant R₂X' de formule : 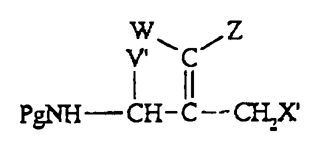 dans laquelle V', W et Z sont H, pour obtenir la 5'-désoxy-5'-(3-amino-2-méthylènepropyl)aminoadénosine.

9. Procédé de préparation d'un composé de formule : 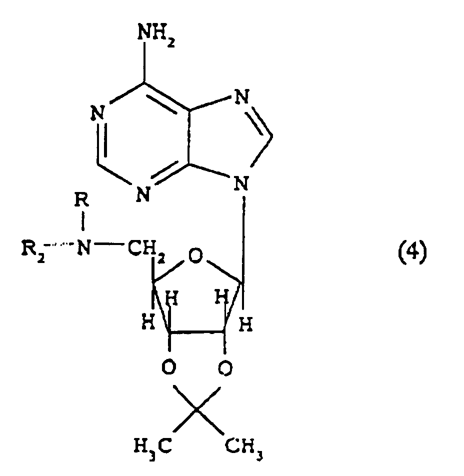 où R₂ est une entité selon l'une des formules : 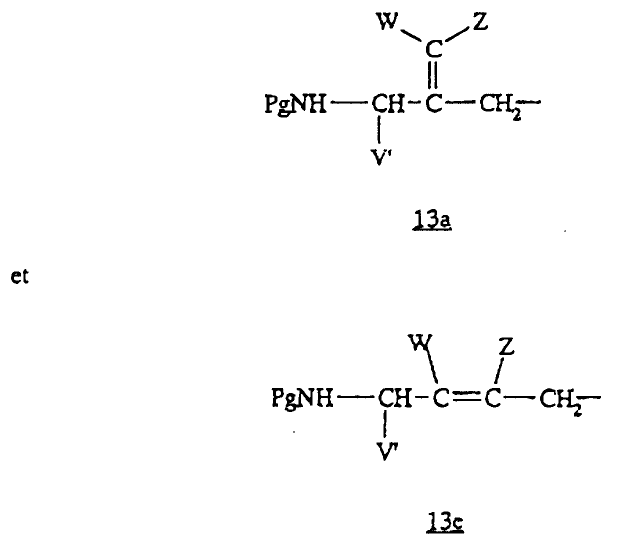 où
V' est H,-COOH ou -COOR₃, R₃ étant une entité du protection réactionnelle ;
W est H, F, Cl ou Br,
Z est H, F, Cl ou Br,
Pg est un groupe N-protecteur,
R est l'hydrogène ou un alkyle en C₁-C₇
qui comprend la réaction d'un composé de formule 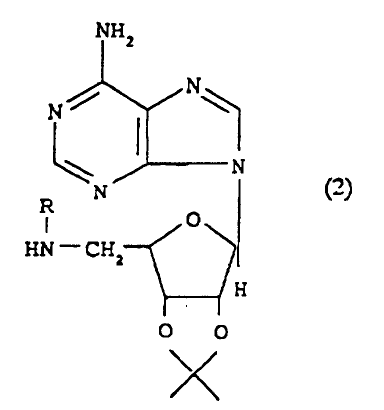 avec un corps réagissant R₂X', R₂X' étant un composé de formule : 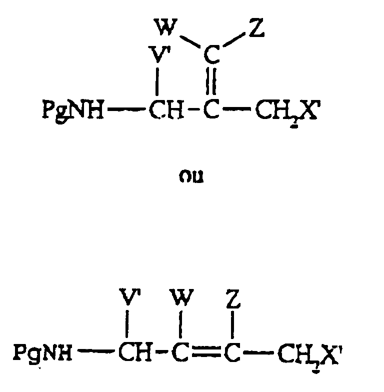 où Pg est une entité N-protectrice, V' est H, COOH ou COOR₃, R₃ étant une entité de protection réactionnelle, et X' est un triflate, chloro, bromo ou iodo, ladite réaction étant conduite avec des quantités équimolaires des corps réagissants impliqués, en présence d'une base, de préférence dans un solvant basique, à des températures d'environ 30°C à 80°C.

10. Utilisation d'un composé selon l'une quelconque des revendications 1 à 9 pour la préparation de compositions pharmaceutiques.

## Revendications (Revendications pour l'(les) Etat(s) contractant(s) suivant(s): GR)

1. Composé de formule : 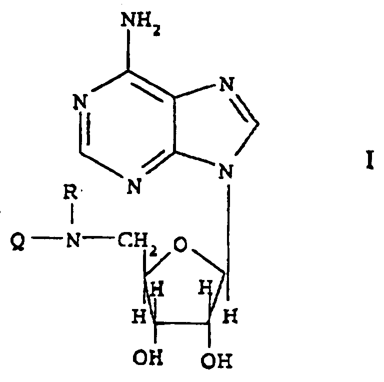 et ses sels pharmaceutiquement acceptables, où
R est l'hydrogène ou un alkyle en C₁-C₇,
Q représente une entité selon l'une des formules Ia ou Ie : 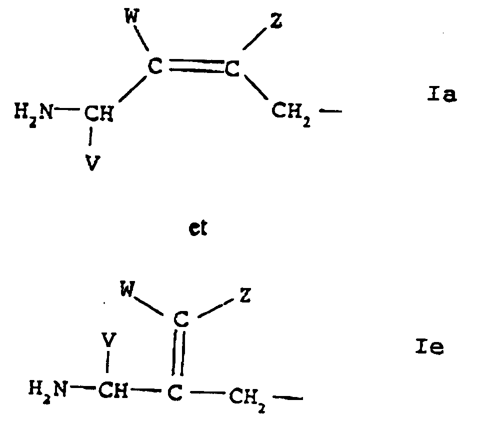 où
V est H ou -COOH,
W est H, F, Cl ou Br,
Z est H, F, Cl ou Br.

2. Composé selon la revendication 1, dans lequel R est méthyle ou H.

3. Composé selon la revendication 1, ledit composé étant la cis-5'-désoxy-5'-(4-amino-2-butényl)méthylaminoadénosine.

4. Composé selon la revendication 1, ledit composé étant la cis-5'-désoxy-5'-(4-amino-2-butényl)aminoadénosine.

5. Composé selon la revendication 1, ledit composé étant la cis-5'-désoxy-5'-(4-amino-4-carboxy-2-butényl)méthylaminoadénosine.

6. Composé selon la revendication 1, ledit composé étant la cis-5'-désoxy-5'-(4-amino-4-carboxy-2-butényl)aminoadénosine.

7. Composé selon la revendication 1, ledit composé étant la 5'-désoxy-5'-(3-amino-2-méthylènepropyl)méthylaminoadénosine.

8. Composé selon la revendication 1, ledit composé étant la 5'-désoxy-5'-(3-amino-2-méthylènepropyl)aminoadénosine.

9. Composé de formule : 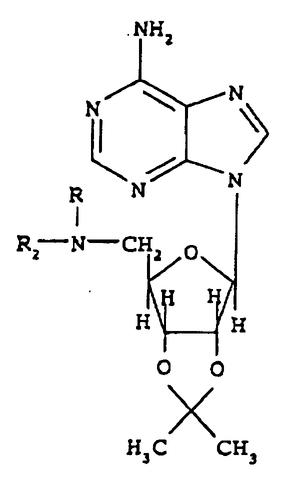 où R₂ est une entité selon l'une des formules : 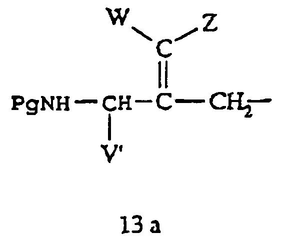 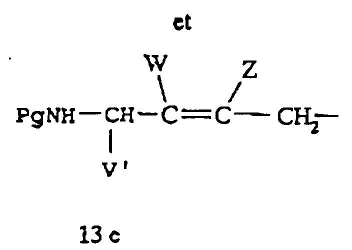 où
V' est H, COOH ou COOR₃, R₃ étant une entité de protection réactionnelle ;
W est H, F, Cl ou Br,
Z est H, F, Cl ou Br,
Pg est un groupe N-protecteur,
R est H ou alkyle en C₁-C₇.

10. Procédé de préparation d'un composé de formule : 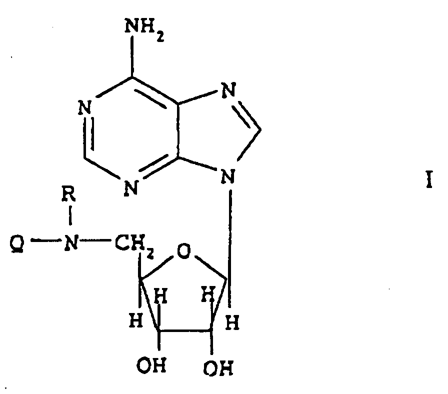 et de ses sels pharmaceutiquement acceptables, où
R est l'hydrogène ou un alkyle en C₁-C₇,
Q représente une entité selon l'une des formules Ia ou Ie : 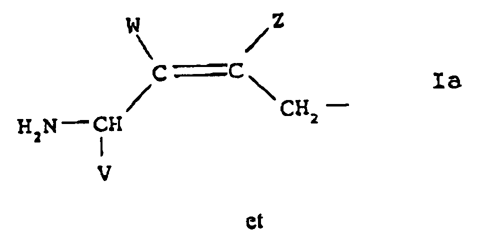 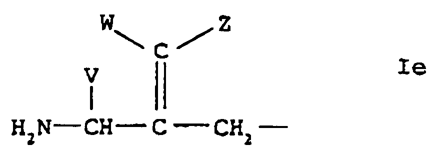 où
V est H ou COOH,
W est H, F, Cl ou Br,
Z est H, F, Cl ou Br,
qui comprend la réaction d'un composé de formule : 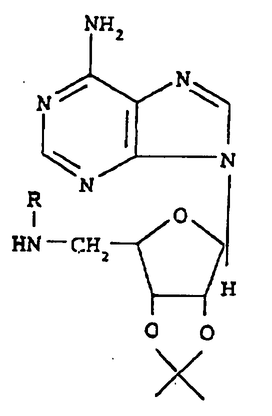 avec un corps réagissant R₂X', R₂X' étant un composé de formule : 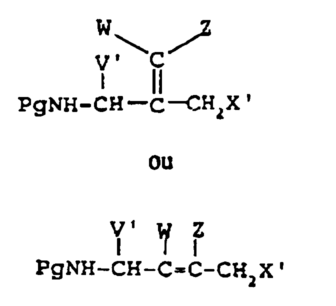 où Pg est une entité N-protectrice, V' est H, COOH ou COOR₃, R₃ étant une entité de protection réactionnelle, et X' est un triflate, chloro, bromo ou iodo, ladite réaction étant conduite avec des quantités équimolaires des corps réagissants impliqués, en présence d'une base, de préférence dans un solvant basique, à des températures d'environ 30°C à 80°C, puis l'élimination de tous les groupes protecteurs à l'aide de techniques standards, et éventuellement la transformation des composés ainsi produits en leurs sels pharmaceutiquement acceptables.

11. Utilisation d'un composé selon l'une quelconque des revendications 1 à 9 pour la préparation de compositions pharmaceutiques.
